# EUROPEAN PATENT APPLICATION

(11) **EP 3 249 396 A1**
(43) Date of publication of application: **29.11.2017**
(21) Application number: 16740169.4
(22) Date of filing: 19.01.2016
(51) Int. Cl.: G01N 27/48, G01N 27/327, G01N 27/416

(54) **METAL ION DETECTION METHOD, ANALYTE DETECTION METHOD, ELECTRODE SUBSTRATE, AND DETECTION KIT**

(30) Priority: 21.01.2015 JP 2015009793
(71) Applicant: Sysmex Corporation, Kobe-shi, Hyogo 651-0073 (JP)
(72) Inventor: HORI, Nobuyasu, Kobe-shi Hyogo 651-0073 (JP); KIRIMURA, Hiroya, Kobe-shi Hyogo 651-0073 (JP); TAKAMURA, Yuzuru, Nomi-shi Ishikawa 923-1292 (JP); CHIKAE, Miyuki, Nomi-shi Ishikawa 923-1292 (JP)
(74) Representative: Hoffmann Eitle
(86) International application number: PCT/JP2016/051451
(87) International publication number: WO 2016/117562

(57) **Abstract**

Provided is a method for detecting a test substance. In this method, metal is deposited or a complex containing a test substance and a metal particle is immobilized on a working electrode on an electrode substrate including the working electrode and a counter electrode. An oxidation potential is applied to the working electrode to generate metal ions, then a reduction potential is applied to a portion having an area smaller than an area of the portion to which an oxidation potential is applied in the working electrode to deposit metal on the surface of the portion to which the reduction potential is applied, and current, voltage or charge caused by the metal deposited is measured to detect metal ions or a test substance.

## Description

### TECHNICAL FIELD

The present invention relates to a method for detecting metal ions, a method for detecting a test substance, an electrode substrate and a detection kit.

### BACKGROUND

As a method for detecting metal ions or other test substances contained in a sample, for example, there is a method using an electrochemical measurement method, and the like (see, for example, Patent Literature 1).

Patent Literature 1 describes a method for measuring silver ions or a test substance with use of an electrochemical measurement method. The method for measuring silver ions described in Patent Literature 1 is performed as follows. First, silver ions contained in a sample containing the silver ions are electrochemically deposited on a working electrode. Next, the presence or absence or concentration of silver ions is measured by measuring current during electrochemical oxidization of the deposited silver. In addition, the method for measuring a test substance described in Patent Literature 1 is performed as follows. First, fine silver particles in an amount corresponding to the amount of a test substance are accumulated in the vicinity of the surface of a working electrode by utilizing biological interaction. Next, the accumulated fine silver particles are electrochemically oxidized and eluted. The eluted silver ions are reduced to deposit silver on the surface of the working electrode. Thereafter, the presence or absence or concentration of the test substance is detected by measuring current during electrochemical oxidization of the deposited silver.

### CITATIONS LIST

Patent Literature 1: JP 2007-190130 A

### SUMMARY OF INVENTION

### TECHNICAL PROBLEMS

The present invention provides a method for detecting metal ions and a method for detecting a test substance, which can detect metal ions or a test substance with high detection sensitivity, an electrode substrate for use in these methods, and a detection kit for a test substance.

### SOLUTIONS TO PROBLEMS

In the method described in Patent Literature 1, the present inventors have found, as will be described later, that when a low concentration of silver ions or a test substance is measured, an S/N ratio (value of a ratio of signal to noise) is small, and the linearity is sometimes poor in a relationship between the signal and the concentration of silver ions or test substance. In addition, the present inventors have applied a reduction potential to a portion having an area smaller than an area of a portion to which an oxidation potential is applied in order to generate metal ions, thereby depositing metal. As a result, the present inventors have found that a large S/N ratio can be obtained even when, for example, the concentration of metal ions or test substance in a sample is low. In addition, the present inventors have found that excellent linearity can be obtained in a relationship between the signal and the concentration of metal ions or test substance, for example, even when the concentration of metal ions or test substance in a sample is low. The present invention has been made based on these findings.

One aspect of the present invention includes a method for detecting a test substance, the method including:
an immobilization step of immobilizing a complex containing a test substance and a metal particle on a surface of a working electrode on an electrode substrate including the working electrode and a counter electrode;
an ionization step of applying an oxidation potential to the working electrode, so that metal ions are generated from the metal particle in the complex immobilized on the working electrode;
a deposition step of applying a reduction potential to a portion having an area smaller than an area of the portion to which an oxidation potential is applied in the working electrode, so that metal formed from the metal ions is deposited on a surface of a portion to which the reduction potential is applied; and
a measurement step of measuring current, voltage or charge caused by the metal deposited in the deposition step.

Other aspects of the present invention include a method for detecting a test substance, the method including:
an immobilization step of immobilizing a complex containing a test substance and a metal particle on a surface of each of a first working electrode and a second working electrode on an electrode substrate including the first working electrode, the second working electrode and a counter electrode;
an ionization step of applying an oxidation potential to the first working electrode and the second working electrode, so that metal ions are generated from the metal particle in the complex immobilized on each of the first working electrode and the second working electrode;
a deposition step of applying a reduction potential to the first working electrode, without applying a reduction potential to the second working electrode, so that metal formed from the metal ions is deposited on a surface of the first working electrode; and
a measurement step of measuring current, voltage or charge caused by the metal deposited in the deposition step.

Still other aspects of the present invention include a method for detecting metal ions, the method including:
a first deposition step of bringing a sample containing metal ions into contact with a surface of a working electrode on an electrode substrate including the working electrode and a counter electrode and applying a reduction potential to the working electrode, so that metal formed from the metal ions is deposited on the surface of the working electrode;
an ionization step of applying an oxidation potential to the working electrode, so that metal ions are generated from the metal deposited in the first deposition step;
a second deposition step of applying a reduction potential to a portion having an area smaller than an area of the portion to which an oxidation potential is applied in the working electrode, so that the metal formed from the metal ions is deposited on a surface of the portion to which the reduction potential is applied; and
a measurement step of measuring current, voltage or charge caused by the metal deposited in the deposition step to measure the current, the voltage or the charge.

Still other aspects of the present invention include a method for detecting metal ions, the method including:
a first deposition step of bringing a sample containing metal ions into contact with a surface of each of working electrodes on an electrode substrate including a first working electrode, a second working electrode and a counter electrode and applying a reduction potential to the working electrode, so that metal formed from the metal ions is deposited on the surface of each of the first working electrode and the second working electrode;
an ionization step of applying an oxidation potential to the first working electrode and the second working electrode, so that metal ions are generated from the metal deposited in the first deposition step;
a second deposition step of applying a reduction potential to the first working electrode, without applying a reduction potential to the second working electrode, so that the metal formed from the metal ions is deposited on the surface of the first working electrode; and
a measurement step of measuring current, voltage or charge caused by the metal deposited in the deposition step to measure the current, the voltage or the charge.

Another aspect of the present invention includes an electrode substrate for use in the method for detecting metal ions or the method for detecting a test substance described above, the electrode substrate including a working electrode and a counter electrode, in which the working electrode includes a portion for generating metal ions and a portion for depositing metal after generation of the metal ions, and is configured so that the portion for depositing the metal after generation of the metal ions is smaller than the portion for generating the metal ions.

Another aspect of the present invention includes an electrode substrate for use in the method for detecting a test substance or the method for detecting metal ions described above, the electrode substrate including a working electrode and a counter electrode, in which the working electrode includes a first working electrode where an oxidation potential is applied and a reduction potential is applied after the application of the oxidation potential, and a second working electrode where an oxidation potential is applied but a reduction potential is not applied after the application of the oxidation potential.

Another aspect of the present invention includes a detection kit for a test substance, the kit including the above-described electrode substrate and a reagent containing metal particles.

### ADVANTAGEOUS EFFECTS OF INVENTION

The present invention can provide a method for detecting metal ions and a method for detecting a test substance, which can detect metal ions or a test substance with high detection sensitivity, an electrode substrate for use in these methods, and a detection kit for a test substance.

### BRIEF DESCRIPTION OF DRAWINGS

FIG. 1 is a perspective view showing a detection device.
FIG. 2 is a block diagram showing a configuration of the detection device shown in FIG. 1.
FIG. 3(A) is a front view showing an example of an electrode substrate, and FIG. 3(B) is a partial enlarged explanatory view of the electrode substrate shown in FIG. 3(A).
FIG. 4(A) is a front view showing a modified example of an electrode substrate, and FIG. 4(B) is a partial enlarged explanatory view of the electrode substrate shown in FIG. 4(A).
FIG. 5(A) is a front view showing a modified example of an electrode substrate, and FIG. 5(B) is a partial enlarged explanatory view of the electrode substrate shown in FIG. 5(A).
FIG. 6 is a step explanatory view showing an example of a treatment procedure of a method for detecting metal ions.
FIG. 7 is a step explanatory view showing a modified example of a treatment procedure of the method for detecting metal ions.
FIG. 8 is a step explanatory view showing an example of a treatment procedure of a method for detecting a test substance.
FIG. 9 is a step explanatory view showing a modified example of a treatment procedure of the method for detecting a test substance.
FIG. 10 is a perspective view showing a detection kit.
FIG. 11 is a graph showing a result of detecting current caused by silver ions contained in a sample, using the electrode substrate of FIG. 3 in Example 1, Comparative Example 1 and Comparative Example 2.
FIG. 12 is a graph showing a result of detecting current caused by a silver nanoparticle in a labeled complex bound to a test substance contained in a sample, using the electrode substrate of FIG. 4 in Example 2, Comparative Example 3 and Comparative Example 4.
FIG. 13 is a graph showing a result of examining a relationship between a concentration of a test substance in a sample and current caused by a silver nanoparticle in a labeled complex bound to the test substance contained in the sample, using the electrode substrate of FIG. 4 in Example 3 and Comparative Example 5.
FIG. 14 is a graph showing a result of examining a relationship between a concentration of a test substance in a sample and current caused by a silver nanoparticle in a labeled complex bound to the test substance contained in the sample, using the electrode substrate of FIG. 3, in Example 4 and Comparative Example 6.
FIG. 15 is a graph showing a result of detecting current caused by a silver nanoparticle in a labeled complex bound to a test substance contained in a sample, using the electrode substrate of FIG. 5 in Example 5 and Comparative Example 7.
FIG. 16 is a graph showing a result of detecting current caused by a silver nanoparticle in a labeled complex bound to a test substance contained in a sample, using the electrode substrate of FIG. 4 in Example 6, Comparative Example 8 and Comparative Example 9.

### DESCRIPTION OF EMBODIMENTS

### 1. Definitions of Terms

The term "X or more" herein includes the value of X and values larger than X. Also, the term "Y or less" includes the value of Y and values smaller than Y. Furthermore, the description of numerical ranges by endpoints, such as "X to Y", includes all numbers and rational numbers contained within each range, and the described endpoints.

The term "test substance" herein means a substance other than metal ions. Examples of the test substance include, but are not particularly limited to, nucleic acids, proteins, peptides, sugar chains, antigens, antibodies, and the like.

### 2. Configuration of Detection Device

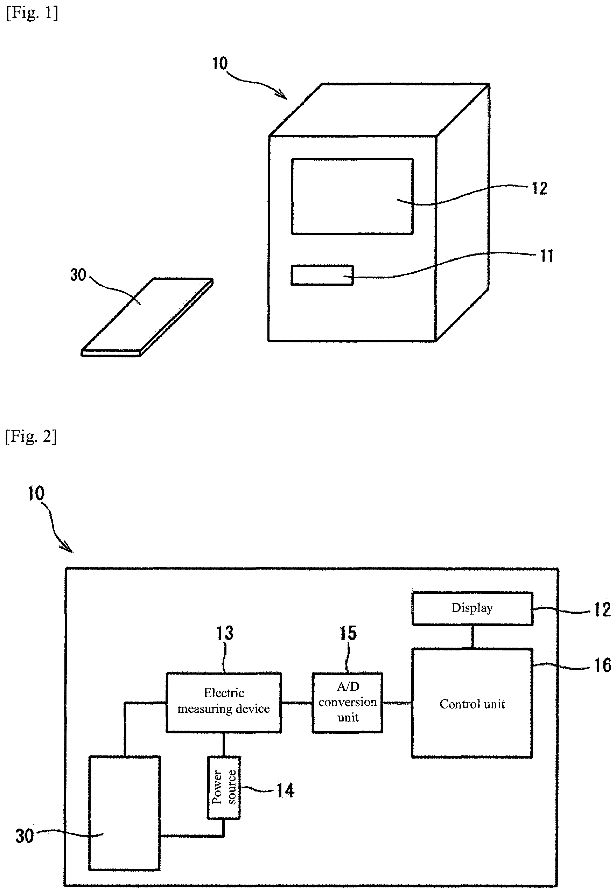

First, an example of a detection device used for the method for detecting metal ions and the method for detecting a test substance will be described with reference to the accompanying drawings. In FIG. 1, a detection device 10 includes a substrate receiving unit 11 into which an electrode substrate 30 is inserted, and a display 12 for displaying a detection result. As shown in FIG. 2, the detection device 10 includes the display 12, an electric measuring device 13, a power source 14, an A/D conversion unit 15, and a control unit 16.

The electric measuring device 13 measures current, voltage or charge caused by metal to be deposited. The power source 14 applies a predetermined potential to an electrode provided on the electrode substrate 30. The A/D conversion unit 15 digitally converts a current value, a voltage value or a charge value measured by the electric measuring device 13. The control unit 16 includes a CPU (Central Processing Unit), a ROM (Read Only Memory), a RAM (Random Access Memory), and the like. The control unit 16 controls operations of the display 12, the electric measuring device 13, and the power source 14. In addition, as described later, the control unit 16 controls application of an oxidation potential and an reduction potential to a working electrode on the electrode substrate. Further, the control unit 16 can approximate the amount of metal, based on a calibration curve indicating the relationship between the current value, the voltage value or the charge value previously prepared from the current value digitally converted by the A/D conversion unit 15 and the amount of metal, and can calculate the amount of metal ions or the amount of a test substance. The display 12 displays information such as the amount of metal ions or the amount of a test substance calculated by the control unit 16.

### 3. Constitution of Electrode Substrate

An electrode substrate according to the present embodiment includes a working electrode and a counter electrode. The working electrode includes a portion for generating metal ions (hereinafter also referred to as "ion generation part") and a portion for depositing metal (hereinafter also referred to as "metal deposition part") after generation of the metal ions. Also, the working electrode is configured so that the area of the metal deposition part is smaller than the area of the ion generation part. Therefore, noise is reduced as compared with a case where metal ion generation and metal deposition after the metal ion generation are performed in the entire working electrode, because measurement of current, voltage or charge is performed in only a part of the working electrodes. Further, according to the electrode substrate of the present embodiment, it is possible to generate metal ions in a large area portion (ion generation part) and then accumulate the metal ions in a small area portion (metal deposition part) to deposit metal. Therefore, it is possible to obtain a signal equivalent to that in a case where metal ion generation and metal deposition after the metal ion generation are performed in the entire working electrode. Therefore, an S/N ratio is improved.

In the working electrode, an oxidation-reduction potential application part and an oxidation potential application part may be provided. An oxidation potential is applied to the oxidation-reduction potential application part for metal ion generation, and a reduction potential is applied for metal deposition after the metal ion generation. An oxidation potential is applied to the oxidation potential application part for generating metal ions, but a reduction potential is not applied after the metal ion generation. The oxidation-reduction potential application part and the oxidation potential application part are electrically insulated from each other. In this case, the ion generation part includes the oxidation-reduction potential application part and the oxidation potential application part. In addition, a metal deposition part includes an oxidation-reduction potential application part.

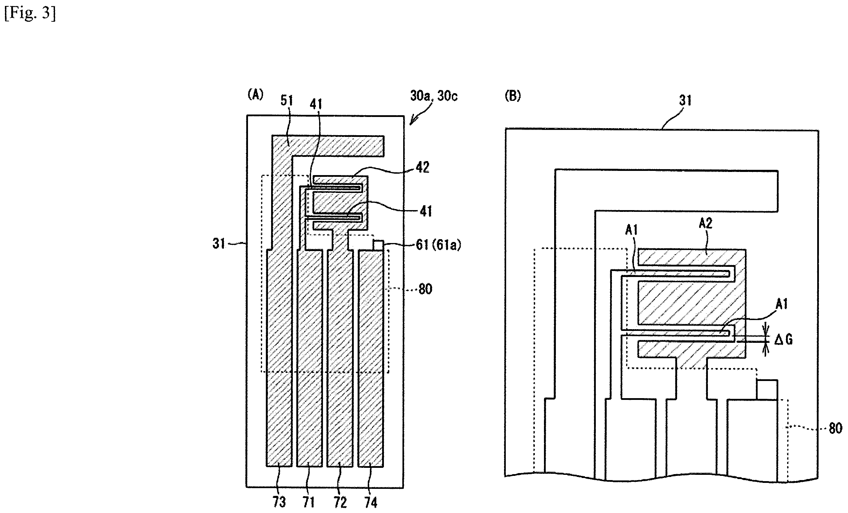

Hereinafter, an example of the electrode substrate will be described with reference to the accompanying drawings. In FIG. 3(A), an electrode substrate 30a includes a substrate main body 31.

On a surface of the substrate main body 31, a first working electrode 41, a second working electrode 42, a counter electrode 51, a reference electrode 61, and electrode leads 71, 72, 73, and 74 are formed. A resist insulating film 80 is arranged so that the first working electrode 41, the second working electrode 42, the counter electrode 51 and the reference electrode 61 are exposed, and a part of the electrode leads 71, 72, 73, and 74 are covered with the resist insulating film 80. Accordingly, contact of a sample with each electrode is maintained, and leakage of the sample to each electrode lead is suppressed. In an embodiment shown in FIG. 3, the first working electrode 41 is integrally constituted with the electrode lead 71. In FIG. 3(B), the first working electrode 41 is an exposed portion that is not covered with the resist insulating film 80. In this portion, deposition of metal, ionization of metal, and the like are performed. In FIG. 3(B), the first working electrode 41 is of two parts denoted by A1. In addition, the second working electrode 42 is integrally constituted with the electrode lead 72. In FIG. 3(B), the second working electrode 42 is an exposed portion that is not covered with the resist insulating film 80. In this portion, ionization of metal, and the like are performed. In FIG. 3(B), the first working electrode 41 and the second working electrode 42 are hatched.

The first working electrode 41 and the second working electrode 42 are arranged on one side part (the upper side in FIG. 3(A)) of the substrate main body 31. The electrode lead 71 extends from the first working electrode 41 toward the other side part (the lower side in FIG. 3(A)) of the substrate main body 31. The electrode lead 72 extends from the second working electrode 42 toward the other side part (the lower side in FIG. 3) of the substrate main body 31. The counter electrode 51 is arranged on an outer side than the first working electrode 41 and the second working electrode 42 (above the first working electrode 41 and the second working electrode 42 in FIG. 3(A)) in the substrate main body 31. The electrode lead 73 extends from the counter electrode 51 toward the other side part (the lower side in FIG. 3) of the substrate main body 31, bypassing the first working electrode 41, the second working electrode 42 and the reference electrode 61. The reference electrode 61 is arranged at a position facing the counter electrode 51, with the first working electrode 41 and the second working electrode 42 interposed therebetween. The electrode lead 74 extends from the reference electrode 61 toward the other side part (the lower side in FIG. 3) of the substrate main body 31. The electrode leads 71, 72, 73, and 74 are arranged in parallel to one another on the other side part (the lower side in FIG. 3) of the substrate main body 31.

In the electrode substrate 30a shown in FIG. 3, each of both the first working electrode 41 and the second working electrode 42 is an ion generation part, and the first working electrode 41 is a metal deposition part. The first working electrode 41 is an oxidation-reduction potential application part. An oxidation potential and a reduction potential after the application of the oxidation potential are applied to the first working electrode 41. The second working electrode 42 is an oxidation potential application part. An oxidation potential is applied to the second working electrode 42, but a reduction potential is not applied thereto after the application of the oxidation potential. As shown in FIG. 3(B), the first working electrode 41 and the second working electrode 42 are electrically insulated from each other by a gap with a distance ΔG.

The present inventors have found that, when measuring current, voltage or charge accompanied with deposition of metal at the detection of metal ions or a test substance, the smaller the area of the portion where metal is deposited, the lower the noise. Therefore, as the ratio of an area of the first working electrode 41 [refer to A1 in FIG. 3(B)] to an area of all working electrodes [refer to A1 + A2 in FIG. 3(B)] [A1/(A1 + A2)] is smaller, the S/N ratio can be improved. From the viewpoint of improving the S/N ratio, A1/(A1 + A2) is usually preferably 1/2 or less, and more preferably 1/10 or less. On the other hand, the lower limit of A1/(A1 + A2) can be properly determined according to the limitation of formation accuracy by the method of forming an electrode on a surface of a substrate main body, the use of an electrode substrate, and the like. The "all working electrodes" mean a concept including both the first working electrode and the second working electrode.

In the present embodiment, as the distance ΔG between the first working electrode 41 and the second working electrode 42 is smaller, the migration time of metal ions to the first working electrode 41 is shortened. Therefore, it is expected to shorten the operation time required to detect metal ions or a test substance. The distance ΔG between the first working electrode 41 and the second working electrode 42 is usually preferably 100 µm or less, and more preferably 50 µm or less, from the viewpoint of shortening the operation time required to detect metal ions or a test substance. On the other hand, the lower limit value of the distance ΔG between the first working electrode 41 and the second working electrode 42 can be properly determined according to the limitation by the method of forming an electrode on a surface of a substrate main body, the use of an electrode substrate, and the like.

In the electrode substrate 30a shown in FIG. 3, the second working electrode 42 has a comb shape. As shown in FIG. 3, the first working electrode 41 is arranged so as to be inserted into gaps of comb teeth of the second working electrode. Accordingly, metal ions can be easily moved between the first working electrode 41 and the second working electrode 42. In addition, its production is easily performed. The shape of each of the first working electrode 41 and the second working electrode 42 may be a polygonal shape, a rectangular shape, a disk shape, an elliptical shape, or the like.

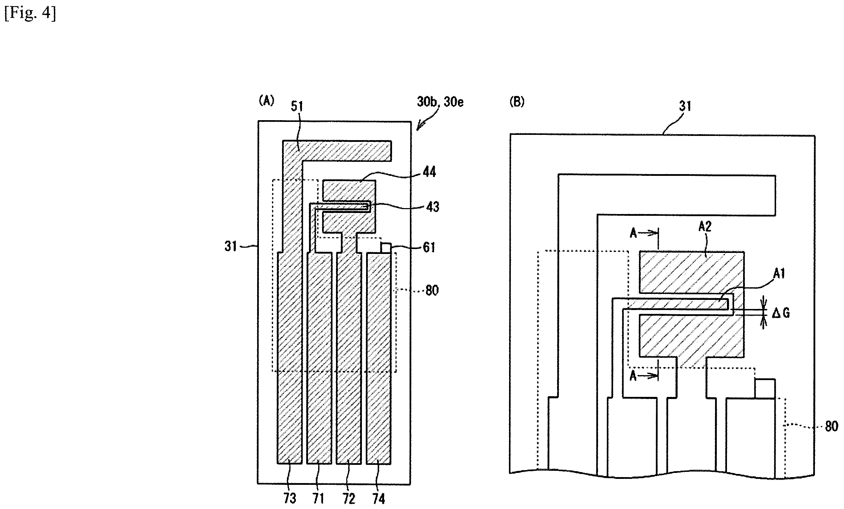

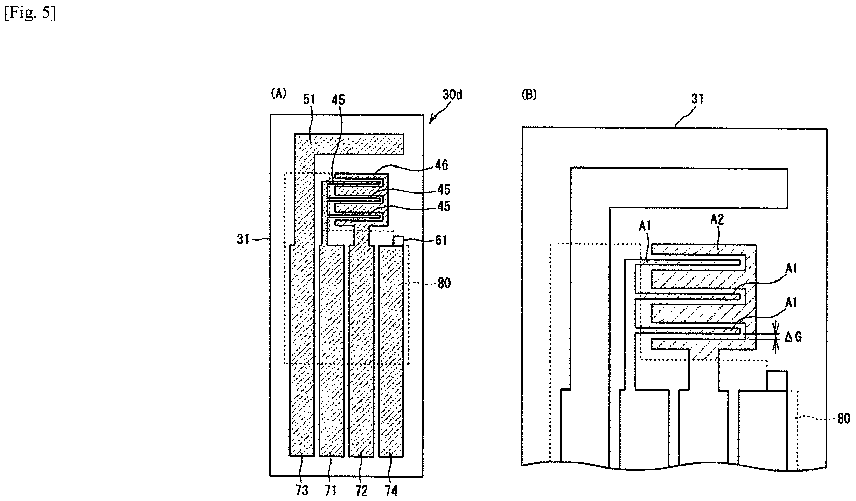

The number of the comb teeth in a comb teeth part of the second working electrode is not particularly limited. In FIG. 3, the number of the comb teeth in the comb teeth part of the second working electrode 42 is three. In FIG. 4, the number of the comb teeth in the comb teeth part of the second working electrode 44 is two. In FIG. 5, the number of the comb teeth in the comb teeth part of the second working electrode 46 is four. The first working electrode denoted by A1 in each of FIG. 3(B), FIG. 4(B) and FIG. 5(B) is preferably arranged so as to be inserted into all gaps of a plurality of the comb teeth of the second working electrode.

One or more electrodes functioning as a first working electrode can be provided on the surface of the substrate main body 31. Further, one or more electrodes functioning as a second working electrode can be provided on the surface of the substrate main body 31.

Each of the first working electrode 41 and the second working electrode 42 includes a thin film having conductivity. The thin film having conductivity mainly includes a conductive material. Examples of the conductive material include, but are not particularly limited to, carbon materials such as graphite, glassy carbon, pyrolytic graphite, carbon paste, carbon fiber, carbon nanotube and graphene; metal materials such as gold and platinum; oxide semiconductors such as indium oxide doped with tin (ITO) and tin oxide doped with fluorine (FTO), and the like. The first working electrode 41 and the second working electrode 42 may include a composite base material or the like in which a conductive layer made of a conductive material is provided on a surface of a nonconductive base material made of a nonconductive material such as glass or plastic. It is desirable that a thickness of the thin film is properly determined according to the use of the electrode substrate and the like.

In FIG. 3, when the electrode substrate is an electrode substrate for use in detection of a test substance, it is preferable that a capture substance that binds to the test substance is immobilized on the surface of each of the first working electrode 41 and the second working electrode 42. Examples of the capture substance include, but are not particularly limited to, nucleic acids, proteins, peptides, sugar chains, antibodies, nanostructures having specific recognition ability, and the like. It is desirable that the capture substance is properly determined according to the kind of the test substance and the like. When the test substance is a nucleic acid, a nucleic acid probe that hybridizes to the nucleic acid, an antibody against the nucleic acid or the like can be used as the capture substance. When the test substance is a protein or a peptide, an antibody can be used as the capture substance. When the test substance is a sugar chain, a lectin for the sugar chain, an antibody against the sugar chain or the like can be used as the capture substance. When the test substance is an antigen, an antibody against the antigen or the like can be used as the capture substance.

It is desirable that an amount of the capture substance per unit area to be immobilized on the surface of each of the first working electrode 41 and the second working electrode 42 is properly determined according to the kind of the test substance, the kind of the capture substance, and the like. The amount of the capture substance per unit area to be immobilized on the surface of each of the first working electrode 41 and the second working electrode 42 may be an amount sufficient to capture the test substance.

In the present embodiment, the substrate main body 31 has a rectangular shape. However, the shape of the substrate main body 31 is not particularly limited, and may be a polygonal shape, a disk shape, or the like.

Examples of the material constituting the substrate main body 31 include, but are not particularly limited to, plastics such as polyethylene terephthalate, polyimide and glass epoxy; inorganic materials such as glass and metals; and the like. It is desirable that the thickness of the substrate main body 31 is properly determined according to the use of the electrode substrate and the like.

The counter electrode 51 comprises a thin film having conductivity. The thin film having conductivity mainly includes a conductive material. The conductive material used for the counter electrode 51 is the same as the conductive material used for the first working electrode 41 and the second working electrode 42. It is desirable that a thickness of the thin film constituting the counter electrode 51 is properly determined according to the use of the electrode substrate and the like.

The reference electrode 61 includes a thin film having conductivity. The thin film having conductivity mainly includes a conductive material. Examples of the conductive material include, but are not particularly limited to, metals such as gold, silver, copper, carbon, platinum, palladium, chromium, aluminum and nickel; alloys including at least one of the metals; metal halides such as metal chlorides; mixtures thereof; and the like. The reference electrode 61 may include a composite base material or the like in which a conductive layer made of a conductive material is provided on a surface of a nonconductive base material made of a nonconductive material such as glass, glass epoxy, or plastic. Specific examples of the reference electrode 61 include, but are not particularly limited to, a silver-silver chloride electrode, a calomel electrode including mercury and mercury chloride, and the like. It is desirable that a thickness of the thin film constituting the reference electrode 61 is properly determined according to the use of the electrode substrate and the like. In this embodiment, the reference electrode 61 is provided on the surface of the substrate main body 31. Therefore, when a large current is measured, the electrode substrate 30a according to the present embodiment can suppress the influence of voltage drop, and can stabilize the voltage applied to the first working electrode 41 and the second working electrode 42. However, in other embodiments, the reference electrode 61 may not be provided on the surface of the substrate main body 31. For example, when a small current with slight influence of voltage drop is measured (for example, 1 µA or less), the counter electrode 51 may serve as the reference electrode 61, although this depends on the kind of the conductive material used for the counter electrode 51, the thickness of the counter electrode 51, and the like.

The electrode substrate according to the present embodiment can be manufactured by, for example, a method including the following steps, or the like. (I) A step of forming a thin film having conductivity so as to form patterns of a working electrode, a counter electrode and optionally a reference electrode on a surface of a substrate main body; (II) a step of immobilizing a capture substance on a surface of the working electrode as necessary; and (III) a step of performing blocking treatment to the surface of the working electrode as necessary.

In the step (I), a thin film having conductivity can be formed, for example, by a method such as a screen printing method or a photolithography method.

When the electrode substrate is used for the detection of a test substance, the step (II) may be performed. In the step (II), the capture substance can be immobilized, for example, via a bonding group that bonds to the conductive material constituting the working electrode. Examples of the bonding group include, but are not particularly limited to, a thiol group, a hydroxyl group, a phosphoric acid group, a carboxyl group, a carbonyl group, an aldehyde group, a sulfonic acid group, an amino group, and the like. Further, the capture substance may be immobilized, for example, by physical adsorption or the like.

When the step (II) is performed, it is desirable to perform the step (III), from the viewpoint of suppressing nonspecific adsorption of a substance other than the test substance (hereinafter, also referred to as "contaminant") to the working electrode. In the step (III), the blocking treatment can be performed by bringing a blocking agent into contact with the working electrode, or the like. The blocking agent may be any blocking agent as long as it suppresses nonspecific adsorption of a contaminant to the working electrode. Examples of the blocking agent include, but are not particularly limited to, solutions prepared by dissolving any one or a plurality of bovine serum albumin, skimmed milk powder, casein and gelatin in a solvent, and the like.

In order to remove excess blocking agent, the obtained electrode substrate can be properly washed to be used.

### 4. Method for Detecting Metal Ions

The method for detecting metal ions according to the present embodiment is a method including:
a first deposition step of bringing a sample containing metal ions into contact with a surface of a working electrode on an electrode substrate including the working electrode and a counter electrode and applying a reduction potential to the working electrode, so that metal formed from the metal ions is deposited on the surface of the working electrode;
an ionization step of applying an oxidation potential to the working electrode, so that metal ions are generated from the metal deposited in the first deposition step;
a second deposition step of applying a reduction potential to a portion having a surface area smaller than a surface area of the portion to which an oxidation potential is applied in the working electrode, so that metal formed from the metal ions is deposited on a surface of a portion to which the reduction potential is applied; and
a measurement step of measuring current, voltage or charge caused by the metal deposited in the deposition step to measure the current, the voltage or the charge (hereinafter, referred to as "method 1").

In method 1 of the present embodiment, a working electrode including a first working electrode and a second working electrode can be used as the working electrode. An oxidation potential and a reduction potential after the application of the oxidation potential are applied to the first working electrode. In addition, an oxidation potential is applied to the second working electrode, but a reduction potential is not applied thereto after the application of the oxidation potential. Each of both the first working electrode and the second working electrode is an ion generation part, and the first working electrode is a metal deposition part. In this case, in method 1 of the present embodiment, metal is deposited on the first working electrode and the second working electrode in the first deposition step. In the ionization step, an oxidation potential is applied to both the first working electrode and the second working electrode. Accordingly, metal ions are generated from the metal deposited on the surface of each of the first working electrode and the second working electrode. Further, in the second deposition step, a reduction potential is not applied to the second working electrode, but a reduction potential is applied to the first working electrode. Accordingly, metal formed from the metal ions is deposited on the surface of the first working electrode. In method 1 according to the present embodiment, for example, the detection device 10 shown in FIG. 1 or the like can be used.

The phrase "detection of metal ions" herein refers to a concept including quantification of metal ions; semi-quantification of metal ions like "strong positive (++)", "weak positive (+)", or "negative (-)"; and qualitative determination of the presence or absence of metal ions.

Examples of the sample containing metal ions include, but are not particularly limited to, river water, seawater, soil, foods, and the like. These samples may be properly subjected to pretreatment before being brought into contact with the working electrode. For example, soil and foods can be dissolved and suspended in an electrolytic solution described later.

Examples of the metal ions include, but are not particularly limited to, transition metal ions such as lead ions, copper ions, mercury ions, cadmium ions, gold ions, platinum ions, silver ions, and zinc ions. According to method 1 according to the present embodiment, among these metal ions, lead ions, copper ions, mercury ions, cadmium ions, gold ions and silver ions can be satisfactorily detected. Transition metal ions can easily deposit or weld metal on the electrode surface by applying a negative potential to the electrode. Therefore, the transition metal ions can be detected by method 1 according to the present embodiment. The metal is metal deposited by reducing metal ions.

In the case of detecting silver ions or other metal ions having properties similar to those of silver ions, current, voltage or charge generated when metal ions are generated from the metal deposited on the surface of the first working electrode in the deposition step is measured in the measurement step. In this case, metal ions can be detected based on the current, the voltage or the charge (hereinafter, referred to as "method 1-1 "). The "other metal ions having properties similar to those of silver ions" are ions that can easily oxidize (ionize) the electrode by applying a positive voltage to the electrode, as with silver ions. Examples of the other metal ions having properties similar to those of silver ions include, but are not particularly limited to, lead ions, copper ions, mercury ions, cadmium ions, zinc ions, and the like.

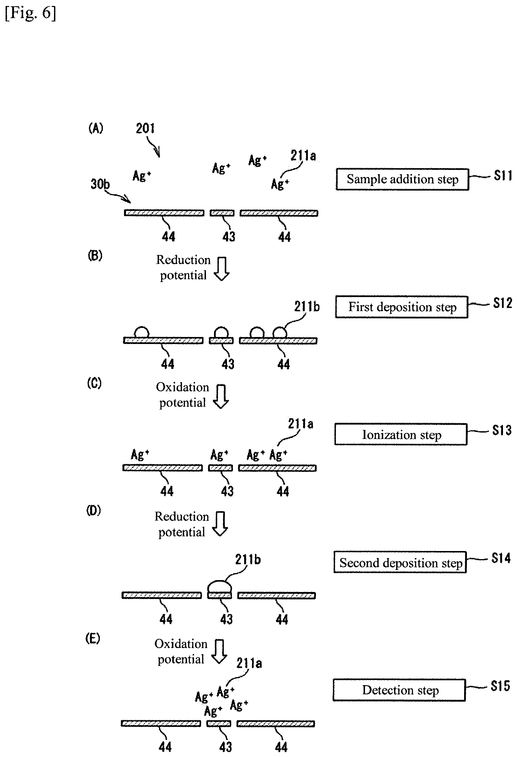

Hereinafter, an example of the treatment procedure of method 1-1 according to the present embodiment will be described with reference to the accompanying drawings. In FIG. 6, the case of detecting silver ions with use of an electrode substrate 30b will be described as an example, the electrode substrate 30b including a first working electrode 43 and a second working electrode 44 formed so as to have the pattern shown in FIG. 4(A) and having no capture substance. FIG. 6 is a cross-sectional explanatory view of the first working electrode 43 and the second working electrode 44 taken along the line A-A in FIG. 4(B). In the embodiment shown in FIG. 4, the first working electrode 43 is integrally constituted with an electrode lead 71. In FIG. 4(B), the first working electrode 43 is an exposed portion that is not covered with a resist insulating film 80. In this portion, deposition of metal, ionization of metal, and the like are performed. In FIG. 4(B), the first working electrode 43 is denoted by A1. In addition, the second working electrode 44 is integrally constituted with an electrode lead 72. In FIG. 4(B), the second working electrode 44 is an exposed portion that is not covered with the resist insulating film 80. In this portion, ionization of the metal and the like are performed. In FIG. 4(B), the first working electrode 43 and the second working electrode 44 are hatched.

First, as shown in FIG. 6(A), a user adds a sample 201 containing silver ions 211 a to an electrode substrate 30b so as to cover respective exposed portions of a first working electrode 43, a second working electrode 44, a counter electrode 51 (not shown) and a reference electrodes 61 (not shown) in a sample addition step S11. Next, the user inserts an electrode substrate 30 into the substrate receiving unit 11 of the detection device 10 shown in FIG. 1. Here, electrode leads 71, 72, 73, and 74 on the electrode substrate 30 inserted into the detection device 10 are connected to the electric measuring device 13 and power source 14 of the detection device 10.

Next, as shown in FIG. 6(B), the user applies a reduction potential to both the first working electrode 43 and the second working electrode 44 in a first deposition step S12. Here, the user instructs the detection device 10 to start a treatment procedure. Accordingly, a predetermined reduction potential based on the reference electrode 61 is applied to both the first working electrode 43 and the second working electrode 44 by the power source 14 of the detection device 10.

The reduction potential is a potential at which metal ions to be detected are reduced to form metal. Therefore, the reduction potential can be properly determined according to the kind of metal ions. When the metal ions are, for example, silver ions, the reduction potential is -1.2 to 0 V based on silver/silver chloride. When the metal ions are, for example, copper ions, the reduction potential is -1.2 to -0 V based on silver/silver chloride. The reduction potential may be kept constant at the application or may be changed with the lapse of time. The application time of the reduction potential can be properly determined according to the use of method 1 of the present embodiment, the kind of the sample, the amount of metal ions contained in the sample, the kind of metal ions, and the like. In the first deposition step S12, a reduction potential is applied to both the first working electrode 43 and the second working electrode 44. Accordingly, as shown in FIG. 6(B), it is possible to reduce the silver ions 211 a in both the first working electrode 43 and the second working electrode 44, and to deposit silver 211b on the surfaces of both the first working electrode 43 and the second working electrode 44. Accordingly, the silver 211b formed from the silver ions 211 a contained in the sample 201 can be collected on the surfaces of both the first working electrode 43 and the second working electrode 44.

After the first deposition step S12 is performed, it is preferable to wash the respective exposed portions of the first working electrode 43, the second working electrode 44, the counter electrode 51 (not shown) and the reference electrode 61 (not shown) with a cleaning liquid. Examples of the cleaning liquid include water; buffer solutions such as phosphate buffered saline and tris buffered saline; electrolytic solutions used in an ionization step S 13, a second deposition step S 14 and a detection step S 15 described later; and the like. Examples of the electrolyte used in the electrolytic solution include, but are not particularly limited to, hydrochloric acid, sodium chloride, potassium chloride, sodium thiocyanate, potassium thiocyanate, and the like. Also, examples of the solvent used in the electrolytic solution include, but are not particularly limited to, water; buffer solutions such as phosphate buffered saline and tris buffered saline; and the like.

Next, as shown in FIG. 6(C), the user drops an electrolytic solution so as to cover the respective exposed portions of the first working electrode 43, the second working electrode 44, the counter electrode 51 and the reference electrode 61, and applies an oxidation potential to both the first working electrode 43 and the second working electrode 44 in the ionization step S13. Here, a predetermined reduction potential based on the reference electrode 61 is applied to both the first working electrode 43 and the second working electrode 44 by the power source 14 of the detection device 10. The electrolytic solution is the same as the electrolytic solution described above.

The oxidation potential is a potential at which metal is oxidized to generate metal ions. Therefore, the oxidation potential can be properly determined according to the kind of metal. When the metal ions are, for example, silver ions, the oxidation potential is 1 to 2.5 V based on silver/silver chloride. When the metal ions are, for example, copper ions, the oxidation potential is 0.8 to 2.0 V based on silver/silver chloride. The oxidation potential may be kept constant at the application or may be changed with the lapse of time. The application time of the oxidation potential can be properly determined according to the use of method 1 of the present embodiment, the amount of metal, the kind of the sample, the kind of metal, and the like. In the ionization step S 13, an oxidation potential is applied to both the first working electrode 43 and the second working electrode 44. Accordingly, as shown in FIG. 6(C), it is possible to oxidize the silver 211b in both the first working electrode 43 and the second working electrode 44, and to generate the silver ions 211 a from the silver 211b in the vicinity of both the first working electrode 43 and the second working electrode 44.

Next, as shown in FIG. 6(D), a reduction potential is not applied to the second working electrode 44, but a reduction potential is applied only to the first working electrode 43 in the second deposition step S 14. Here, a predetermined reduction potential based on the reference electrode 61 is applied only to the first working electrode 43 by the power source 14 of the detection device 10.

The reduction potential and the application time in the second deposition step S 14 are the same as the reduction potential and the application time in the first deposition step S 12. The reduction potential is applied in the presence of an electrolytic solution. The electrolytic solution used in the second deposition step S14 is the same as the electrolytic solution used in the ionization step S 13. In the second deposition step S14, a potential may be applied within a range in which the silver 211b is not deposited on the second working electrode 44. As described above, a reduction potential is applied to a portion having an area smaller than an area of the portion to which an oxidation potential is applied in order to generate the silver ions 211a in the ionization step S 13, so that the silver 211b is deposited in the second deposition step S14. Accordingly, as shown in FIG. 6(D), the silver 211b formed from the silver ions 211 a generated in the ionization step can be accumulated and deposited in the first working electrode 43. Therefore, it is possible to obtain a signal equivalent to that in a case where metal ion generation and metal deposition after the metal ion generation are performed in the entire working electrode.

Thereafter, as shown in FIG. 6(E), the user measures in the detection step S 15 current, voltage or charge generated when the silver 211b on the surface of the first working electrode 43 is ionized, as the current, the voltage or the charge caused by the silver 211b on the surface of the first working electrode 43 in the second deposition step S 14. Here, a predetermined oxidation potential based on the reference electrode 61 is applied only to the first working electrode 43 by the power source 14 of the detection device 10. Also, simultaneously with the application, the current, the voltage or the charge between the first working electrode 43 and the counter electrode 51 caused by the silver 211b is measured by the electric measuring device 13.

The oxidation potential is applied in the presence of an electrolytic solution. The electrolytic solution used in the detection step S15 is the same as the electrolytic solution used in the ionization step S 13.

In the detection step S 15, examples of the method of measuring the current, the voltage or the charge include, but are not particularly limited to, a differential pulse voltammetry method, a cyclic voltammetry method, a chronoamperometry method, and the like.

In the case of quantifying metal ions by method 1-1, for example, an amount of metal ions contained in a sample can be obtained based on a calibration curve prepared using a sample containing a known amount of metal ions. The calibration curve can be prepared by plotting the relationship between the current, the voltage or the charge caused by the metal measured by performing each step of method 1-1 using a sample containing a known amount of metal ions and the amount of metal ions. Further, in the case of semi-quantifying metal ions by method 1-1, for example, the use of a plurality of samples containing known amounts of metal ions and a plurality of samples containing no metal ion can give not only positive and negative determination based on the concentration of metal ions, but also further classification of the samples determined as positive. For example, by setting a threshold for separating "strong positive (++)" and "weak positive (+)" as well as a threshold separating "weak positive (+)" and "negative (-)", "strong positive (++)", "weak positive (+)" or "negative (-)" can be determined. In the case of qualitatively determining the presence or absence of metal ions by method 1-1, it can be determined that "the sample contains metal ions" when a change in current, voltage or charge is observed with respect to the current, the voltage or the charge obtained using a sample containing no metal ion. On the other hand, it can be determined that "the sample does not contain metal ions" when no change in current, voltage or charge is observed with respect to the current, the voltage or the charge obtained using a sample containing no metal ion.

As described above, the surface area of the first working electrode 43 used for measuring the current, the voltage or the charge caused by the silver 211b is smaller than the surface area of all working electrodes. Therefore, in method 1-1 according to the present embodiment, measurement of current, voltage or charge is performed in only a part of the working electrodes. Accordingly, noise is reduced, as compared with a case where metal ion generation and metal deposition after the metal ion generation are performed in the entire working electrode. Therefore, method 1-1 according to the present embodiment can give a high S/N ratio even when, for example, the concentration of silver ions in the sample is low. Moreover, method 1-1 according to the present embodiment can give excellent linearity in the relationship between the silver ion concentration and the corresponding signal at low concentration. Therefore, method 1-1 according to the present embodiment can detect metal ions such as silver ions with high detection sensitivity.

### 5. Modified Example of Method for Detecting Metal Ions

In the case of detecting gold ions or other metal ions having properties similar to those of gold ions, current, voltage or charge until metal is deposited on the first working electrode in the deposition step is measured in the measurement step, and metal ions can be detected based on the application time of a reduction potential until a change in current, a change in voltage or a change in charge accompanied with deposition of metal on the first working electrode is caused (hereinafter referred to as "method 1-2"). The "other metal ions having properties similar to those of gold ions" are metal ions that hardly oxidize a welded metal and require a large voltage, as with gold ions. Examples of the other metal ions having properties similar to those of gold ions include, but are not limited to, platinum ions and the like.

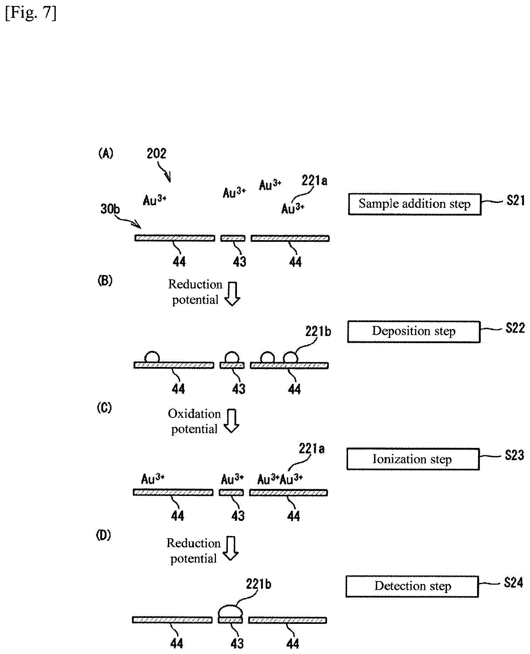

Hereinafter, an example of the treatment procedure of method 1-2 according to the present embodiment will be described with reference to the accompanying drawings. Also in FIG. 7, the case of detecting gold ions with use of an electrode substrate 30b will be described as an example, the electrode substrate 30b including a first working electrode 43 and a second working electrode 44 formed so as to have the pattern shown in FIG. 4(A) and having no capture substance. FIG. 7 is a cross-sectional explanatory view of the first working electrode 43 and the second working electrode 44 taken along the line A-A in FIG. 4(B).

Method 1-2 according to the present embodiment can be performed by the treatment procedure similar to that of method 1-1, except for the following points. 1) In method 1-1, the sample 201 containing the silver ions 211a is used, whereas in method 1-2, a sample 202 containing gold ions 221a is used. 2) In method 1-1, the current, the voltage or the charge when the silver 211b deposited on the surface of the first working electrode 43 is ionized again is measured, and the silver ions 211 a are detected based on the current, the voltage or the charge. On the other hand, in method 1-2, current, voltage or charge when gold 221b is deposited on the surface of the first working electrode 43 is measured, or a change in current, a change in voltage or a change in charge accompanied with the deposition of the gold 221b is measured, and then the gold ions 221 a are detected based on the current, the voltage or the charge upon the deposition of the gold 221b, or the change in current, the change in voltage or the change in charge accompanied with the deposition of the gold 221b.

In method 1-2 according to the present embodiment, first, as shown in FIG. 7(A), a user adds a sample 202 containing gold ions 221 a to an electrode substrate 30b so as to cover respective exposed portions of a first working electrode 43, a second working electrode 44, a counter electrode 51 (not shown) and a reference electrodes 61 (not shown) in a sample addition step S21.

Next, as shown in FIG. 7(B), the user applies a reduction potential to both the first working electrode 43 and the second working electrode 44 in a deposition step S22.

When the metal ions are, for example, gold ions, the reduction potential is -1.0 to 0.5 V based on silver/silver chloride. When the metal ions are, for example, platinum ions, the reduction potential is -1.0 to 0.2 V based on silver/silver chloride. The application time of the reduction potential can be properly determined according to the use of method 1-2 of the present embodiment, the kind of the sample, the kind of metal ions, and the like. In the deposition step S22, a reduction potential is applied to both the first working electrode 43 and the second working electrode 44. Accordingly, as shown in FIG. 7(B), the gold 221b formed from the gold ions 221a contained in the sample 202 can be collected on the surfaces of both the first working electrode 43 and the second working electrode 44.

After the deposition step S22 is performed, it is preferable to wash the respective exposed portions of the first working electrode 43, the second working electrode 44, the counter electrode 51 (not shown) and the reference electrode 61 (not shown) with a cleaning liquid. The cleaning liquid used for the washing is the same as the cleaning liquid used in method 1-1.

Next, as shown in FIG. 7(C), the user applies an oxidation potential to both the first working electrode 43 and the second working electrode 44 in an ionization step S23.

When the metal ions are, for example, gold ions, the oxidation potential is 1.3 to 2.4 V based on silver/silver chloride. When the metal ions are, for example, platinum ions, the oxidation potential is 1.0 to 2.0 V based on silver/silver chloride. The application time of the oxidation potential can be properly determined according to the use of method 1-2 of the present embodiment, the kind of the sample, the kind of metal, and the like. In the ionization step S23, an oxidation potential is applied to both the first working electrode 43 and the second working electrode 44. Accordingly, as shown in FIG. 7(C), it is possible to oxidize the gold 221b in both the first working electrode 43 and the second working electrode 44, and to generate the gold ions 221a from the gold 221b in the vicinity of both the first working electrode 43 and the second working electrode 44.

The oxidation potential is applied in the presence of an electrolytic solution. The electrolytic solution is the same as the electrolytic solution used in method 1-1.

Thereafter, as shown in FIG. 7(D), a reduction potential is not applied to the second working electrode 44, but a reduction potential is applied only to the first working electrode 43 in a detection step S24. Here, a predetermined reduction potential based on the reference electrode 61 is applied only to the first working electrode 43 by the power source 14 of the detection device 10. Also, simultaneously with the application, the current, the voltage or the charge when the gold 221b is deposited on the surface of the first working electrode 43 is measured, or a change in current, a change in voltage or a change in charge accompanied with the deposition of the gold 221b on the surface of the first working electrode 43 is measured, by the electric measuring device 13. Thereafter, the gold ions 221 a are detected based on the current, the voltage or the charge upon deposition of the gold 221b on the first working electrode, or the change in current, the change in voltage or the change in charge accompanied with the deposition of the gold 221b.

The reduction potential and the application time in the detection step S24 are the same as the reduction potential and the application time in the deposition step S22. As described above, a reduction potential is applied to a portion having an area smaller than an area of the portion to which an oxidation potential is applied in order to generate the gold ions 221a in the ionization step S23, so that the gold 221b is deposited in the detection step S24. Therefore, as shown in FIG. 7(D), the gold 221b formed from the gold ions 221a generated in the ionization step can be accumulated and deposited in the first working electrode 43.

The reduction potential is applied in the presence of an electrolytic solution. The electrolytic solution is the same as the electrolytic solution used in method 1-1.

In the detection step S24, the current, the voltage and the charge upon deposition of the gold 221b, as well as the change in current, the change in voltage and the change in charge accompanied with the deposition of the gold 221b can be measured by the same method as the method of measuring the current, the voltage or the charge in the detection step S15 in method 1-1. For the detection of the gold ions 221 a, for example, the current amount or charge amount flowed until the gold 221b is deposited on the surface of the first working electrode 43, the voltage necessary for the deposition of the gold 221b, the time required until the current value depending on the deposition is saturated, and the like can be used as indexes.

In method 1-2, quantification of the metal ions, semi-quantification of the metal ions and qualitative determination of the presence or absence of the metal ions can be carried out in the same manner as in method 1-1.

As described above, the surface area of the first working electrode 43 used for measuring the current, the voltage or the charge caused by the gold 221b is smaller than the surface area of all working electrodes. Therefore, according to method 1-2 of the present embodiment, it is possible to detect metal ions such as gold ions with high detection sensitivity, as in method 1-1.

### 6. Method for Detecting Test Substance

The method for detecting a test substance according to the present embodiment includes:
an immobilization step of immobilizing a complex containing a test substance and a metal particle on a surface of a working electrode on an electrode substrate including the working electrode and a counter electrode;
an ionization step of applying an oxidation potential to the working electrode, so that metal ions are generated from the metal particle in the complex immobilized on the working electrode;
a deposition step of applying a reduction potential to a portion having an area smaller than an area of the portion to which an oxidation potential is applied in the working electrode, so that metal formed from the metal ions is deposited on a surface of a portion to which the reduction potential is applied; and
a measurement step of measuring current, voltage or charge caused by the metal deposited in the deposition step (hereinafter referred to as "method 2").

In method 2 of the present embodiment, a working electrode including a first working electrode and a second working electrode can be used as the working electrode. An oxidation potential and a reduction potential after the application of the oxidation potential are applied to the first working electrode. In addition, an oxidation potential is applied to the second working electrode, but a reduction potential is not applied thereto after the application of the oxidation potential. Each of both the first working electrode and the second working electrode is an ion generation part, and the first working electrode is a metal deposition part. In this case, in method 2 of the present embodiment, a complex is immobilized on the surface of each of the first working electrode and the second working electrode in the immobilization step. In the ionization step, an oxidation potential is applied to both the first working electrode and the second working electrode, so that metal ions are generated from the metal particle in the complex immobilized on each of the first working electrode and the second working electrode. Further, in the deposition step, a reduction potential is not applied to the second working electrode, but a reduction potential is applied to the first working electrode, so that metal formed from the metal ions is deposited on the surface of the first working electrode. In method 2 according to the present embodiment, for example, the detection device 10 shown in FIG. 1 or the like can be used.

The phrase "detection of a test substance" herein refers to a concept including quantification of a test substance; semi-quantification of a test substance like "strong positive (++)", "weak positive (+)", or "negative (-)"; and qualitative determination of the presence or absence of a test substance.

Examples of the sample containing a test substance include, but are not particularly limited to, specimens typified by whole blood, plasma, serum, urine, saliva, and the like; river water, seawater, soil, foods, and the like.

Examples of the metal particles include, but are not particularly limited to, transition metal particles such as gold particles, silver particles, copper particles, lead particles, platinum particles and indium particles; composite particles of respective metals such as gold-silver composite particles and gold-copper composite particles; quantum dots (also referred to as "semiconductor particles") such as cadmium sulfide and zinc sulfide; and the like. The particle diameter of the metal particle is preferably 5 nm or more, and more preferably 10 nm or more, from the viewpoint of sufficiently obtaining signals of current, voltage and electrical charge upon deposition of metal. Further, the particle diameter of the metal particle is preferably 200 nm or less, and more preferably 100 nm or less, from the viewpoint of improving the capture efficiency of the test substance and the labeling efficiency of the test substance. The term "metal particle" herein includes not only a spherical metal particle but also a metal particle having a shape other than a spherical shape. The term "particle diameter" refers to a diameter when the metal particle has a spherical shape, and refers to an average of the maximum diameter and the minimum diameter of the particles when the metal particle has a shape other than a spherical shape. The particle diameter of the metal particle can be determined by, for example, a light scattering method, microscopic observation, or the like.

In method 2 according to the present embodiment, in a method using particles of silver, particles of other metals having properties similar to those of silver as the metal particles, or quantum dots (hereinafter also referred to as "method 2-1 "), current, voltage or charge generated when metal ions are generated from the metal deposited on the surface of the first working electrode in the deposition step is measured in the measurement step. In this case, the test substance is detected based on the current, the voltage or the charge. The "other metals having properties similar to those of silver" are metals that can be easily formed into particles and that can easily oxidize (ionize) the electrode by applying an oxidation potential to the electrode, as with silver. Examples of the metals having properties similar to those of silver include, but are not particularly limited to, copper, lead, indium, and the like. When quantum dots are used in method 2-1, it is possible to measure current, voltage or charge generated when metal ions are generated from the metal obtained from metal ions constituting the quantum dots. For example, when the quantum dot is cadmium sulfide, it is possible to measure current, voltage or charge generated when cadmium ions are generated from cadmium. Further, for example, when the quantum dot is zinc sulfide, it is possible to measure current, voltage or charge generated when zinc ions are generated from zinc.

In method 2 according to the present embodiment, in a method using particles of gold or particles of other metals having properties similar to those of gold as the metal particles (hereinafter also referred to as "method 2-2"), current, voltage or charge until metal is deposited on the first working electrode in the deposition step is measured in the measurement step. In this case, the test substance is detected based on the application time of a reduction potential until a change in current, a change in voltage or a change in charge accompanied with deposition of the metal on the first working electrode is caused. The "other metals having properties similar to those of gold" are metals that can be formed into particles and that require a high oxidation potential when oxidized on the electrode, as with gold. Examples of the other metals having properties similar to those of gold include, but are not particularly limited to, platinum and the like.

When composite particles of respective metals such as gold-silver composite particles and gold-copper composite particles are used in method 2, either method 2-1 or method 2-2 can be performed according to the kind of the metal constituting the composite particles.

It is preferable that a binding substance that binds to the test substance is immobilized on the metal particle. It is preferable that the binding substance binds to a site different from the site to which a capture substance binds in the test substance. The binding substance is appropriately selected according to the kind of the test substance, or the like. When the test substance is a nucleic acid, a nucleic acid probe that hybridizes to the nucleic acid, an antibody against the nucleic acid, a protein that binds to the nucleic acid, or the like can be used as the binding substance. When the test substance is a protein or a peptide, an antibody against the protein, an antibody against the peptide, or the like can be used as the binding substance. When the test substance is a sugar chain, a lectin for the sugar chain, an antibody against the sugar chain, or the like can be used as the binding substance.

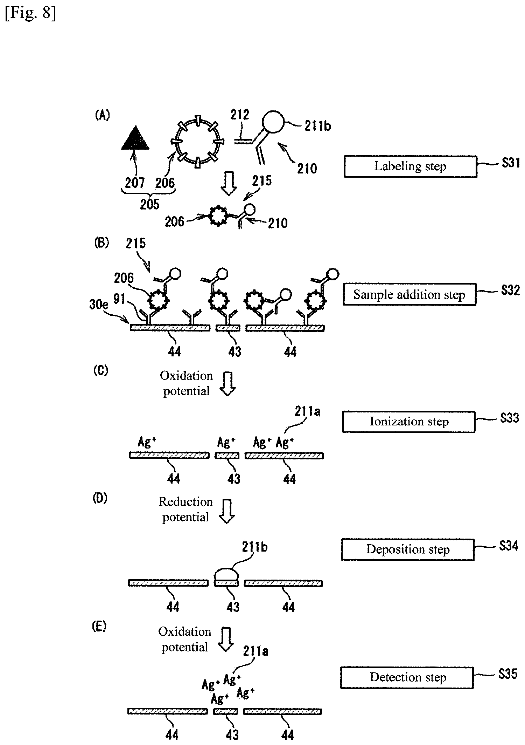

Hereinafter, an example of the treatment procedure of method 2-1 will be described with reference to the accompanying drawings. In FIG. 8, the case of using silver as metal ions with use of a labeling binding substance 210 on which a binding substance 212 that binds to a test substance 206 is immobilized on a silver particle 211b, and an electrode substrate 30e will be described as an example, the electrode substrate 30e including a first working electrode 43 and a second working electrode 44 formed so as to have the pattern shown in FIG. 4(A) and having a capture substance. FIG. 8 is a cross-sectional explanatory view of the first working electrode 43 and the second working electrode 44 taken along the line A-A in FIG. 4(B). In the present embodiment, a labeled complex 215 containing the test substance 206 is immobilized on the surface of each of the first working electrode 43 and the second working electrode 44 using a biological interaction between a capture substance 91 and the test substance 206 on each of the first working electrode 43 and the second working electrode 44. Examples of the biological interaction include, but are not particularly limited to, antigen-antibody reactions, hydrogen bonds between nucleic acids, bonds between nucleic acids and nucleic acid binding proteins, bonds between lectins and sugar chains, and the like. The capture substance 91 is the same as the capture substance mentioned in the description of the constitution of the electrode substrate.

First, as shown in FIG. 8(A), a user mixes a sample 205 containing a test substance 206 with a labeling binding substance 210, and labels the test substance 206 with the labeling binding substance 210 in a labeling step S31. Accordingly, a labeled complex 215 containing the test substance 206 and the labeling binding substance 210 is formed. In the figure, a reference numeral 207 denotes a contaminant possibly included in the sample 205.

The labeling binding substance 210 includes a binding substance 212 that binds to the test substance 206 and silver 211b serving as a labeling substance.

In the labeling step S31, an amount of the labeling binding substance 210 to the sample 205 can be properly determined according to the kind of the sample 205 and the like. Usually, it is desirable that the amount of the labeling binding substance 210 to the sample 205 is an excess amount to an amount of the test substance 206 expected to be contained in the sample 205.

Next, as shown in FIG. 8(B), the user adds a sample containing the labeled complex 215 to an electrode substrate 30e so as to cover respective exposed portions of a first working electrode 43 and a second working electrode 44 in a sample addition step S32. Then, the user inserts the electrode substrate 30e into the substrate receiving unit 11 of the detection device 10 shown in FIG. 1. Here, electrode leads 71, 72, 73, and 74 on the electrode substrate 30e inserted in the detection device 10 are connected to the electric measuring device 13 and power source 14 of the detection device 10.

In the sample addition step S32, the test substance 206 of the labeled complex 215 contained in the sample is captured by the capture substance 91 on the surface of each of the first working electrode 43 and the second working electrode 44 formed on a substrate main body 31 of the electrode substrate 30e. Accordingly, the labeled complex 215 containing the test substance 206 and the silver 211b is immobilized on the surface of each of the first working electrode 43 and the second working electrode 44. The contaminant 207 in the sample is not captured by the capture substance 91.

In the present embodiment, as shown in FIG. 8(A), the labeling step S31 is performed before the sample is added to the electrode substrate 30e. Accordingly, the labeled complex 215 containing the test substance 206 in the sample 205 and the labeling binding substance 210 is formed. Thereafter, a sample containing the obtained labeled complex 215 is brought into contact with the capture substance 91 on the surface of each of the first working electrode 43 and the working electrode 44. However, the order of contact is not particularly limited among the sample 205 containing the test substance 206, the labeling binding substance 210, and the capture substance 91 on the surface of each of the first working electrode 43 and the working electrode 44. For example, first, the capture substance 91 on the surface of each of the first working electrode 43 and the working electrode 44 may be brought into contact with the sample 205 containing the test substance 206. In this case, after the contact between the capture substance 91 and the sample 205, the test substance 206 captured by the capture substance 91 and the labeling binding substance 210 may be brought into contact with each other. On the other hand, first, the labeling binding substance 210 may be added to the electrode substrate 30e so as to cover the respective exposed portions of the first working electrode 43 and the second working electrode 44. In this case, after the addition, the sample 205 containing the test substance 206 may be added.

In the sample addition step S32, the capture of the test substance 206 in the labeled complex 215 by the capture substance 91 can be performed, for example, under a condition that the capture substance 91 and the test substance 206 are bound with each other. The condition that the capture substance 91 and the test substance 206 are bound with each other can be appropriately selected according to the kind of the test substance 206 and the like. When the test substance 206 is a nucleic acid and the capture substance 91 is a nucleic acid probe that hybridizes to the nucleic acid, the capture of the test substance 206 can be performed in the presence of a hybridization buffer. When the capture substance 91 is an antibody or an antigen, it is preferable to perform the capture of the test substance 206 in a solution suitable for causing an antigen-antibody reaction. Examples of the solution for performing an antigen-antibody reaction include, but are not particularly limited to, phosphate buffered saline, HEPES buffer, PIPES buffer, Tris buffer, and the like. When the test substance 206 is a sugar chain and the capture substance 91 is a lectin for the sugar chain, it is preferable that the test substance 206 binds to the capture substance 91 in a solution suitable for binding of the sugar chain to the lectin. In this case, examples of the solution for binding of the sugar chain to the lectin include, but are not particularly limited to, buffer solutions such as phosphate buffered saline and tris buffered saline; aqueous solutions in which salt concentration is adjusted with a salt such as sodium chloride or potassium chloride; and the like.

In the present embodiment, the immobilization of the labeled complex 215 on the surface of each of the first working electrode 43 and the second working electrode 44 can be performed by the biological interaction between the test substance 206 and the capture substance 91.

After the sample addition step S32 is performed, it is preferable to wash respective exposed portions of the first working electrode 43, the second working electrode 44, a counter electrode 51 (not shown) and a reference electrode 61 (not shown) with a cleaning liquid. The cleaning liquid used for the washing is the same as the cleaning liquid used in methods 1-1 and 1-2.

Next, as shown in FIG. 8(C), the user applies an oxidation potential to both the first working electrode 43 and the second working electrode 44 in an ionization step S33. Here, a predetermined reduction potential based on the reference electrode 61 is applied to both the first working electrode 43 and the second working electrode 44 by the power source 14 of the detection device 10.

The oxidation potential and the application time of the oxidation potential in the ionization step S33 are a potential at which metal is oxidized to generate metal ions as in methods 1-1 and 1-2. Therefore, the oxidation potential can be properly determined according to the kind of metal. As shown in FIG. 8(C), by applying the oxidation potential to both the first working electrode 43 and the second working electrode 44 in the ionization step S33, it is possible to oxidize the silver 211b in the labeled complex 215 in both the first working electrode 43 and the second working electrode 44, and to generate the silver ions 211 a in the vicinity of both the first working electrode 43 and the second working electrode 44.

The oxidation potential is applied in the presence of an electrolytic solution. The electrolytic solution is the same as the electrolytic solution used in method 1-1 and method 1-2.

Next, as shown in FIG. 8(D), the user does not apply a reduction potential to the second working electrode 44, but applies a reduction potential only to the first working electrode 43 in a deposition step S34. Here, a predetermined reduction potential based on the reference electrode 61 is applied only to the first working electrode 43 by the power source 14 of the detection device 10.

The reduction potential and the application time in the deposition step S34 are the same as the reduction potential and the application time in method 1-1 and method 1-2. In the deposition step S34, a potential may be applied within a range in which the silver 211b is not deposited on the second working electrode 44. As described above, in the deposition step S34, a reduction potential is applied to a portion having an area smaller than an area of the portion to which an oxidation potential is applied in order to generate the silver ions 211 a in the ionization step S33, so that the silver 211b is deposited. Accordingly, as shown in FIG. 8(D), the silver 211b formed from the silver ions 211 a generated in the ionization step can be accumulated and deposited in the first working electrode 43.

The reduction potential is applied in the presence of an electrolytic solution. The electrolytic solution is the same as the electrolytic solution used in method 1-1 and method 1-2.

Thereafter, as shown in FIG. 8(E), the user measures current, voltage or charge generated when the silver 211b on the surface of the first working electrode 43 is ionized in a detection step S35, as the current, the voltage or the charge caused by the silver 211b on the surface of the first working electrode 43 in the deposition step S34. Here, a predetermined oxidation potential based on the reference electrode 61 is applied only to the first working electrode 43 by the power source 14 of the detection device 10. Also, simultaneously with the application, the current, the voltage or the charge between the first working electrode 43 and the counter electrode caused by the silver 211b is measured by the electric measuring device 13.

The oxidation potential is applied in the presence of an electrolytic solution. The electrolytic solution is the same as the electrolytic solution used in method 1-1 and method 1-2.

In the detection step S35, the method of measuring current, voltage or charge is the same as the method of measuring current, voltage or charge in method 1-1.

In method 2-1, quantification of the test substance, semi-quantification of the test substance and qualitative determination of the presence or absence of the test substance can be carried out in the same manner as in methods 1-1 and 1-2.

As described above, the surface area of the first working electrode 43 used for measuring the current, the voltage or the charge caused by the silver 211b is smaller than the surface area of all working electrodes. Therefore, in method 2-1 according to the present embodiment, measurement of current, voltage or charge is performed in only a part of the working electrodes. Accordingly, noise is reduced, as compared with a case where metal ion generation and metal deposition after the metal ion generation are performed in the entire working electrode. Therefore, method 2-1 according to the present embodiment can give a high S/N ratio even when, for example, the concentration of the test substance in the sample is low. Further, method 2-1 according to the present embodiment can give excellent linearity in the relationship between the signal based on silver proportional to the amount of the test substance in the low concentration region and the concentration of the test substance. Therefore, method 2-1 according to the present embodiment can detect a test substance with high detection sensitivity.

### 7. Modified Example of Method for Detecting Test Substance

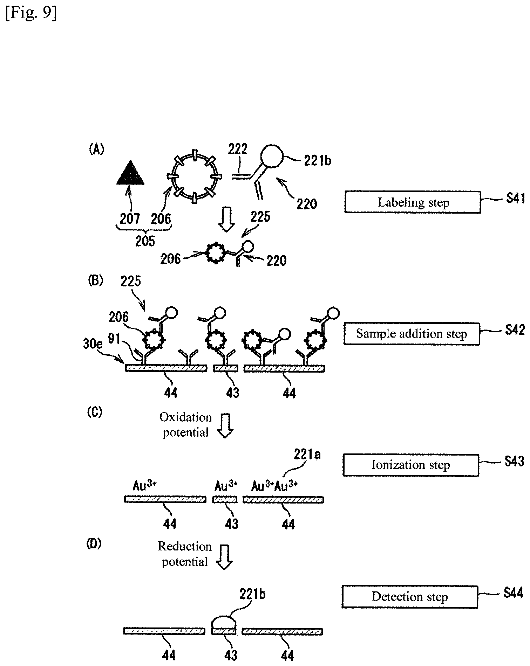

Next, an example of the treatment procedure of method 2-2 will be described with reference to the accompanying drawings. In FIG. 9, the case of detecting gold with use of a labeling binding substance 220 on which a binding substance 222 that binds to a test substance 206 is immobilized on a gold particle 221b, and an electrode substrate 30e will be described as an example, the electrode substrate 30e including a first working electrode 43 and a second working electrode 44 formed so as to have the pattern shown in FIG. 4(A) and having a capture substance. FIG. 9 is a cross-sectional explanatory view of the first working electrode 43 and the second working electrode 44 taken along the line A-A in FIG. 4(B). In the present embodiment, a labeled complex 215 containing the test substance 206 is immobilized on the surface of each of the first working electrode 43 and the second working electrode 44 using a biological interaction between a capture substance 91 and the test substance 206 on each of the first working electrode 43 and the second working electrode 44.

Method 2-2 according to the present embodiment can be performed by the treatment procedure similar to that of method 2-1, except for the following points. 1) In method 2-1, the labeling binding substance 210 containing the silver particle serving as the silver 211b is used, whereas in method 2-2, the labeling binding substance 220 containing a gold particle serving as the gold 221b is used. 2) In method 2-1, the current, the voltage or the charge when the silver 211b deposited on the surface of the first working electrode 43 is ionized again is measured, and the test substance 206 is detected based on the current, the voltage or the charge. On the other hand, in method 2-2, current, voltage or charge when the gold 221b is deposited on the surface of the first working electrode 43 is measured, or a change in current, a change in voltage or a change in charge accompanied with the deposition of the gold 221b is measured, and then the test substance 206 is detected based on the current, the voltage or the charge upon deposition of the gold 221b, or the change in current, the change in voltage or the change in charge accompanied with the deposition of the gold 221b.

First, as shown in FIG. 9(A), a user mixes a sample 205 containing a test substance 206 with a labeling binding substance 220, and labels the test substance 206 with the labeling binding substance 220 in a labeling step S41. In the figure, a reference numeral 207 denotes a contaminant possibly included in the sample 205.

Next, as shown in FIG. 9(B), the user adds a sample containing a labeled complex 225 to an electrode substrate 30e so as to cover respective exposed portions of a first working electrode 43, a second working electrode 44, a counter electrode 51 (not shown) and a reference electrodes 61 (not shown) in a sample addition step S42.

In the sample addition step S42, the test substance 206 of the labeled complex 225 contained in the sample is captured by the capture substance 91 on the surface of each of the first working electrode 43 and the second working electrode 44 formed on the substrate main body 31 of the electrode substrate 30e. Accordingly, the labeled complex 225 containing the test substance 206 and the gold 221b is immobilized on the surface of each of the first working electrode 43 and the second working electrode 44.

Also in the present embodiment, the order of contact is not particularly limited among the sample 205, the labeling binding substance 220, and the capture substance 91.

In the sample addition step S42, the capture of the test substance 206 in the labeled complex 225 by the capture substance 91 can be performed under the same condition as the capture of the test substance 206 in the sample addition step S32 in method 2-1. In the present embodiment, the immobilization of the labeled complex 225 on the surface of each of the first working electrode 43 and the second working electrode 44 can be performed by the biological interaction between the test substance 206 and the capture substance 91.

After the sample addition step S42 is performed, it is preferable to wash the respective exposed portions of the first working electrode 43, the second working electrode 44, the counter electrode 51 (not shown) and the reference electrode 61 (not shown) with a cleaning liquid. The cleaning liquid used for the washing is the same as the cleaning liquid used in methods 1-1, 1-2 and 2-1.

Next, as shown in FIG. 9(C), the user applies an oxidation potential to both the first working electrode 43 and the second working electrode 44 in an ionization step S43.

The oxidation potential and the application time of the oxidation potential in the ionization step S43 are a potential at which metal is oxidized to generate metal ions, which is similar to the oxidation potential in methods 1-1, 1-2 and method 2-1. Accordingly, as shown in FIG. 9(C), it is possible to oxidize the gold 221b in the labeled complex 225 in both the first working electrode 43 and the second working electrode 44, and to generate the gold ions 221 a in the vicinity of both the first working electrode 43 and the second working electrode 44.

The oxidation potential is applied in the presence of an electrolytic solution. The electrolytic solution used in the ionization step S43 is the same as the electrolytic solution used in methods 1-1, 1-2 and 2-1.

Thereafter, as shown in FIG. 9(D), a reduction potential is not applied to the second working electrode 44, but a reduction potential is applied only to the first working electrode 43 in a detection step S44. Here, a predetermined reduction potential based on the reference electrode 61 is applied only to the first working electrode 43 by the power source 14 of the detection device 10. Also, simultaneously with the application, the current, the voltage or the charge when the gold 221b is deposited on the surface of the first working electrode 43 is measured, or a change in current, a change in voltage or a change in charge accompanied with the deposition of the gold 221b on the surface of the first working electrode 43 is measured, by the electric measuring device 13. Thereafter, the test substance 206 is detected based on the current, the voltage or the charge upon deposition of the gold 221b on the first working electrode, or the change in current, the change in voltage or the change in charge accompanied with the deposition of the gold 221b.

The reduction potential and the application time in the detection step S44 are the same as the reduction potential and the application time in method 1-2. As described above, a reduction potential is applied to a portion having an area smaller than an area of the portion to which an oxidation potential is applied in order to generate the gold ions 221 a in the ionization step S23, so that the gold 221b is deposited in the detection step S24. Also, simultaneously with the application, the current, the voltage or the charge is measured. Therefore, as shown in FIG. 9(D), the gold 221b formed from the gold ions 221a generated in the ionization step can be accumulated and deposited in the first working electrode 43.

The reduction potential is applied in the presence of an electrolytic solution. The electrolytic solution used in the detection step S44 is the same as the electrolytic solution used in methods 1-1, 1-2 and 2-1.

In the detection step S24, the current, the voltage and the charge upon deposition of the gold 221b, as well as the change in current, the change in voltage and the change in charge accompanied with the deposition of the gold 221b can be measured by the same method as the method of measuring the current, the voltage or charge in methods 1-1, 1-2 and 2-1.

In method 2-2, quantification of the test substance, semi-quantification of the test substance and qualitative determination of the presence or absence of the test substance can be carried out in the same manner as in method 2-1.

### 8. Detection Kit for Test Substance

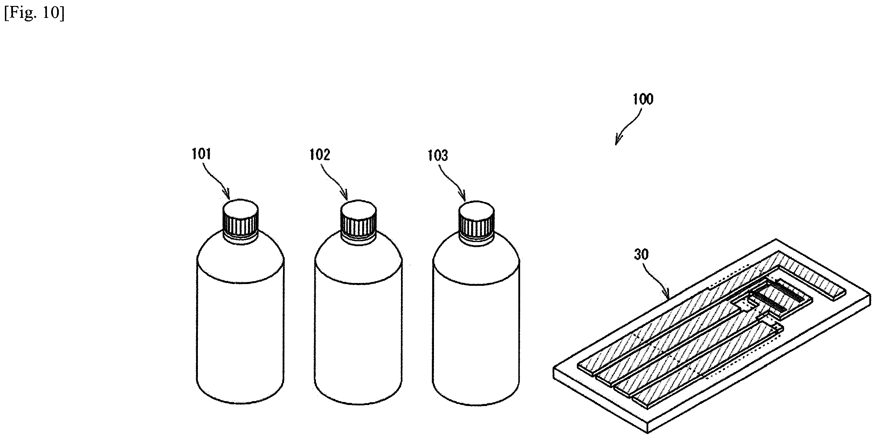

The detection kit for a test substance according to the present embodiment includes the above-described electrode substrate and a reagent containing metal particles. As shown in FIG. 10, the detection kit for a test substance according to the present embodiment can be provided, for example, as a kit 100 including an electrode substrate 30, a reagent bottle 101 containing a metal particle suspension containing metal particles, a reagent bottle 102 containing an electrolytic solution used in application of a reduction potential or an oxidation potential, and a reagent bottle 103 containing a cleaning liquid for washing each electrode. The electrode substrate 30 and the reagent bottles 101, 102, and 103 may be separately provided, or two or more of them may be provided in combination.

Examples of the electrode substrate 30 include, but are not particularly limited to, electrode substrates 30a, 30b, 30c, 30d, and the like.

A solvent used in the metal particle suspension may be any solvent as long as it can stably retain the metal particles. Examples of the solvent used in the metal particle suspension include, but are not particularly limited to, water; buffers such as PBS and TBS containing biomolecules such as bovine serum albumin; aqueous solutions adjusted to low concentrations of salts such as sodium chloride and potassium chloride; and the like. A content of the metal particles in the metal particle suspension can be properly determined according to the use of the detection kit for a test substance, or the like. The electrolytic solution and the cleaning liquid are the same as those used in methods 1-1, 1-2, 2-1 and 2-2.

### EXAMPLES

Hereinafter, the present invention will be described in more detail by way of examples and the like, but the present invention is not limited only to these examples.

### (Example 1) Method for Detecting Silver Ions (1) Preparation of Electrode Substrate

A carbon paste was applied to a surface of a substrate main body 31 made of glass epoxy so as to have the patterns shown in FIG. 3(A), and then dried, thereby forming a first working electrode 41, a second working electrode 42, a counter electrode 51, a reference electrode main body 61a and electrode leads 71, 72, 73, and 74. Next, silver/silver chloride was applied to the tip of the reference electrode main body 61 a to obtain a reference electrode 61. For the purpose of maintaining contact of a sample with each electrode and preventing leakage of the sample to each electrode lead, a resist insulating film [see 80 in FIG. 3(A)] was arranged so that the first working electrode 41, the second working electrode 42, the counter electrode 51 and the reference electrode 61 were exposed and a part of each of the electrode leads 71, 72, 73, 74 was covered, and thus an electrode substrate 30a was obtained.

An area of the first working electrode 41 [A1 in FIG. 3(A)] was set to 0.3 mm². Also, an area of the second working electrode 42 [A2 in FIG. 3(A)] was set to 2.7 mm². Accordingly, [area A1 of first working electrode]/[area of all working electrodes (A1 + A2)] was set to 1/10. A distance between the first working electrode 41 and the second working electrode 42 was set to 50 µm.

### (2) Deposition of Silver

The electrode substrate 30a obtained in Example 1 (1) was connected to a potentiostat (trade name: 832B, manufactured by BAS Inc.). Next, 30 µL of a sample [an aqueous sodium nitrate solution (0 pM silver nitrate) or an aqueous sodium nitrate solution containing 100 pM silver nitrate] was added dropwise so that the first working electrode 41, the second working electrode 42, the counter electrode 51 and the reference electrode 61 were covered. After completion of the dropwise addition, a potential of -1.0 V based on the reference electrode 61 was applied to both the first working electrode 41 and the second working electrode 42 for 300 seconds, and silver was deposited on the surface of each of the first working electrode 41 and the second working electrode 42.

Next, the liquid on the surface of each of the first working electrode 41, the second working electrode 42, the counter electrode 51 and the reference electrode 61 was removed. Thereafter, the first working electrode 41, the second working electrode 42, the counter electrode 51 and the reference electrode 61 were washed with purified water and then air-dried.

### (3) Measurement of Silver Ions

The electrode substrate 30a on which silver was deposited in Example 1 (2) was connected to a potentiostat. Next, 30 µL of a 0.05 M aqueous sodium chloride solution was added dropwise so that the first working electrode 41, the second working electrode 42, the counter electrode 51 and the reference electrode 61 were covered. After completion of the dropwise addition, a potential of 2.1 V based on the reference electrode 61 was applied to both the first working electrode 41 and the second working electrode 42 for 30 seconds. Thereafter, a potential of -1.0 V based on the reference electrode 61 was applied only to the first working electrode 41 for 300 seconds. Thereafter, with respect to the first working electrode 41, the potential was swept between -0.4 V and 0.4 V by differential pulse voltammetry method, and the current flowing between the first working electrode 41 and the counter electrode 51 was measured.

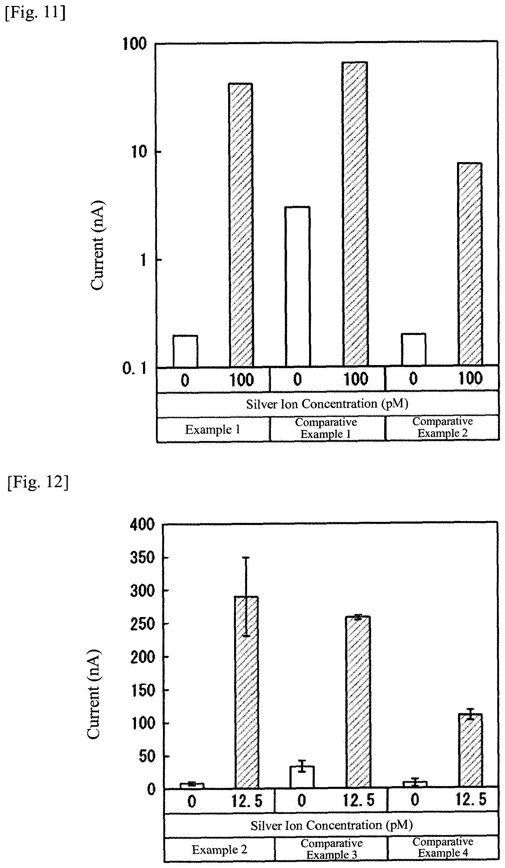

The results of detecting the currents caused by the silver ions contained in the sample in Example 1 are shown in FIG. 11. In the figure, each of white bars indicates a current when the aqueous sodium nitrate solution (0 pM silver nitrate) was used as the sample, and each of hatched bars indicates a current when the aqueous sodium nitrate solution containing 100 pM silver nitrate was used as the sample.

In FIG. 11, an S/N ratio was determined during performing the method for detecting silver ions in Example 1 from the current when the aqueous sodium nitrate solution (0 pM silver nitrate) was used as the sample and the current when the aqueous sodium nitrate solution containing 100 pM silver nitrate was used as the sample. As a result, the S/N ratio was 208.

### (Comparative Example 1) Method for Detecting Silver Ions

The electrode substrate 30a on which silver was deposited in Example 1 (2) was connected to a potentiostat. Next, 30 µL of a 0.05 M aqueous sodium chloride solution was added dropwise so that the first working electrode 41, the second working electrode 42, the counter electrode 51 and the reference electrode 61 were covered. After completion of the dropwise addition, a potential of 2.1 V based on the reference electrode 61 was applied to both the first working electrode 41 and the second working electrode 42 for 30 seconds. Thereafter, a potential of -1.0 V based on the reference electrode 61 was applied to both the first working electrode 41 and the second working electrode 42 for 300 seconds. Thereafter, with respect to each of the first working electrode 41 and the second working electrode 42, the potential was swept between -0.4 V and 0.4 V by differential pulse voltammetry method, and the current flowing between the first working electrode 41 and the counter electrode 51 and the current flowing between the second working electrode 42 and the counter electrode 51 were measured.

The results of detecting the currents caused by the silver ions contained in the sample in Comparative Example 1 are shown in FIG. 11. In FIG. 11, the current detected by the method for detecting a silver ion in Comparative Example 1 is the total of the current flowing between the first working electrode 41 and the counter electrode 51 and the current flowing between the second working electrode 42 and the counter electrode 51. In the figure, each of white bars indicates a current when the aqueous sodium nitrate solution (0 pM silver nitrate) was used as the sample, and each of hatched bars indicates a current when the aqueous sodium nitrate solution containing 100 pM silver nitrate was used as the sample.

In FIG. 11, an S/N ratio was determined during performing the method for detecting silver ions in Comparative Example 1 from the current when the aqueous sodium nitrate solution (0 pM silver nitrate) was used as the sample and the current when the aqueous sodium nitrate solution containing 100 pM silver nitrate was used as the sample. As a result, the S/N ratio was 21.

In the present comparative example, the same electrode substrate as in Example 1 was used. However, in the present comparative example, silver was deposited and ionized at both the first working electrode and the second working electrode. Therefore, the method for detecting silver ions in the present comparative example can be considered as being equivalent to a conventional method in which silver is deposited and ionized at only one working electrode having the total area of the area of the first working electrode and the area of the second working electrode. From the results of Example 1 and Comparative Example 1, it was found that, according to the method in Example 1, a signal equivalent to that of the conventional method is secured, and noise can be reduced as compared with the conventional method.

### (Comparative Example 2) Method for Detecting Silver Ions

The electrode substrate 30a on which silver was deposited in Example 1 (2) was connected to a potentiostat. Next, 30 µL of a 0.05 M aqueous sodium chloride solution was added dropwise so that the first working electrode 41, the second working electrode 42, the counter electrode 51 and the reference electrode 61 were covered. After completion of the dropwise addition, a potential of 2.1 V based on the reference electrode 61 was applied only to the first working electrode for 30 seconds. Thereafter, a potential of -1.0 V based on the reference electrode 61 was applied only to the first working electrode 41 for 300 seconds. Thereafter, with respect to the first working electrode 41, the potential was swept between -0.4 V and 0.4 V by differential pulse voltammetry method, and the current flowing between the first working electrode 41 and the counter electrode 51 was measured.

The results of detecting the currents caused by the silver ions contained in the sample in Comparative Example 2 are shown in FIG. 11. In the figure, each of white bars indicates a current when the aqueous sodium nitrate solution (0 pM silver nitrate) was used as the sample, and each of hatched bars indicates a current when the aqueous sodium nitrate solution containing 100 pM silver nitrate was used as the sample.

In FIG. 11, an S/N ratio was determined during performing the method for detecting silver ions in Comparative Example 2 from the current when the aqueous sodium nitrate solution (0 pM silver nitrate) was used as the sample and the current when the aqueous sodium nitrate solution containing 100 pM silver nitrate was used as the sample. As a result, the S/N ratio was 37.

In the present comparative example, the same electrode substrate as in Example 1 was used. However, in the present comparative example, silver was deposited and ionized at only the first working electrode. Therefore, the method for detecting silver ions in the present comparative example can be considered as being equivalent to a conventional method in which silver is deposited and ionized at only one working electrode having the same area as the area of the first working electrode. From the results of Example 1 and Comparative Example 2, it was found that, according to the method in Example 1, noise is suppressed to be equal to that of Comparative Example 2, and a signal higher than that of Comparative Example 2 is obtained.

From the above results, it was found that, according to the method in Example 1, an S/N ratio higher than that in the methods in Comparative Example 1 and Comparative Example 2 is obtained.

### (Example 2) Method for Detecting Test Substance (1) Preparation of Electrode Substrate Main Body

An electrode main substrate body having the first working electrode 43, the second working electrode 44, the counter electrode 51, and the reference electrode 61 was obtained in same operation as in Example 1(1), except for applying a carbon paste to a surface of the substrate main body 31 so as to have the patterns shown in FIG. 4(A), instead of applying the carbon paste to the surface of the substrate main body 31 so as to have the patterns shown in FIG. 3. An area of the first working electrode 43 [A1 in FIG. 4(B)] was set to 0.3 mm². An area of the second working electrode 44 (A2 in FIG. 4(B)) was set to 2.7 mm². Accordingly, [area A1 of first working electrode]/[area of all working electrodes (A1 + A2)] was set to 1/10. A distance between the first working electrode 43 and the second working electrode 44 was set to 50 µm.

### (2) Immobilization of Primary Antibody

An anti-HBs antigen primary antibody solution (prepared by Sysmex Corporation) was added to a sodium carbonate buffer (pH 9) so that the anti-HBs antigen primary antibody had a concentration of 100 µg/mL to obtain a primary antibody solution. Three µL of the resulting primary antibody solution was added dropwise onto the surface of each of the first working electrode 43 and the second working electrode 44 on the electrode substrate main body obtained in Example 2 (1). Thereafter, the electrode substrate main body was allowed to stand still at room temperature (25°C) for 2 hours under high humidity conditions [measurement range (60% by volume) or more]. Next, the solution on the surface of each of the first working electrode 43 and the second working electrode 44 was removed. Four µL of phosphate buffered saline was added dropwise onto the surface of each of the first working electrode 43 and the second working electrode 44, and washing was performed by pipetting. After the washing, the phosphate buffered saline on the surface of each of the first working electrode 43 and the second working electrode 44 was removed.

### (3) Blocking Treatment

Four µL of 10% (w/w) BSA-containing phosphate buffered saline was added dropwise onto the surface of each of the first working electrode 43 and the second working electrode 44 on the electrode substrate main body to which the primary antibody was immobilized in Example 2 (2). Thereafter, the antibody-immobilized electrode substrate was allowed to stand still overnight at 4°C under high humidity conditions. Next, the solution on the surface of each of the first working electrode 43 and the second working electrode 44 was removed. Four µL of phosphate buffered saline was added dropwise onto the surface of each of the first working electrode 43 and the second working electrode 44, and washing was performed by pipetting. After the washing, the phosphate buffered saline on the surface of each of the first working electrode 43 and the second working electrode 44 was removed to obtain an electrode substrate 30b.

### (4) Preparation of Labeled Complex

An anti-HBs antigen secondary antibody solution (manufactured by Sysmex Corporation) was added to a sodium phosphate buffer (pH 7) so that the anti-HBs antigen secondary antibody had a concentration of 100 µg/mL to obtain a secondary antibody solution.

The pH of the silver nanoparticle solution (manufactured by Sigma-Aldrich, silver nanoparticle diameter: 60 nm, silver nanoparticle concentration: 0.02 mg/mL) was adjusted to 6.2 with use of 1 M hydrochloric acid. Then, 10.1 µL of a 10% (w/v) polyoxyethylene (20) sorbitan monolaurate (Tween 20 (registered trademark)) solution was added to 900 µL of the pH-adjusted silver nanoparticle solution. One-hundred µL of the secondary antibody solution was added to the resulting mixed solution, and the mixture was allowed to stand at room temperature for 20 minutes. Next, 400 µL of a 1% (w/w) BSA-containing TBS-T solution (hereinafter also referred to as "BSA/TBS-T solution") was added to the resulting mixture solution. Thereafter, the resulting mixture solution was subjected to centrifugation at 6500 × g at 4°C for 20 minutes, and the supernatant was removed. To the resulting residue was added 1 mL of a 1 % BSA/TBS-T solution. The resulting mixture solution was subjected to centrifugation at 6500 × g at 4°C for 20 minutes, and the supernatant was removed. To the resulting residue was added 1 mL of a 1% BSA/TBS-T solution. The operation of the centrifugation and removal of the supernatant was further performed two more times. Three-hundred µL of phosphate buffered saline containing 1% (w/w) BSA and 0.05% (w/v) Tween 20 (registered trademark) was added to the resulting residue to obtain a solution containing the labeled complex.

### (5) Immobilization of Test Substance

Fetal bovine serum (hereinafter also referred to as "FBS") was added so that an HBs antigen serving as a test substance (manufactured by Sysmex Corporation, trade name: HISCL HBsAg calibrator, 2500 IU/mL) had a concentration of 12.5 IU/mL to obtain HBs antigen-containing FBS. Next, FBS (concentration of HBs antigen: 0 IU/mL) or the HBs antigen-containing FBS serving as a sample was mixed with the solution containing the labeled complex obtained in Example 2 (2) so that [sample]/[labeled complex solution] (volume ratio) was 3/1.

Four µL of the resulting mixed solution was added dropwise onto the surface of each of the first working electrode 43 and the second working electrode 44 on the electrode substrate 30b obtained in Example 2 (3). Thereafter, the electrode substrate 30b was allowed to stand still at room temperature for 45 minutes under high humidity conditions, thereby causing an antigen-antibody reaction. Next, the solution on the surface of each of the first working electrode 43 and the second working electrode 44 was removed. Four µL of phosphate buffered saline was added dropwise onto the surface of each of the first working electrode 43 and the second working electrode 44, and washing was performed by pipetting. After the washing, the phosphate buffered saline on the surface of each of the first working electrode 43 and the second working electrode 44 was removed. Each of the first working electrode 43 and the second working electrode 44 was washed with use of 1.5 mL of purified water and then air-dried. Accordingly, the test substance was immobilized on each of the first working electrode 43 and the second working electrode 44 on the electrode substrate 30b.

### (6) Electrochemical Measurement

The electrode substrate 30b on which the test substance was immobilized in Example 2 (5) was connected to a potentiostat. Next, 30 µL of a 0.05 M aqueous hydrochloric acid solution was added dropwise so that the first working electrode 43, the second working electrode 44, the counter electrode 51 and the reference electrode 61 were covered. After completion of the dropwise addition, a potential of 2.1 V based on the reference electrode 61 was applied to both the first working electrode 43 and the second working electrode 44 for 15 seconds. Thereafter, a potential of -1.0 V based on the reference electrode 61 was applied only to the first working electrode 43 for 8 minutes. Thereafter, with respect to the first working electrode 43, the potential was swept between -0.4 V and 0.4 V by differential pulse voltammetry method, and the current flowing between the first working electrode 43 and the counter electrode 51 was measured.

The results of detecting the currents caused by the silver nanoparticle in the labeled complex bound to the test substance contained in the sample in Example 2 are shown in FIG. 12. In the figure, each of white bars indicates a current when FBS (concentration of HBs antigen: 0 IU/mL) was used as the sample, and each of hatched bars indicates a current when FBS containing 12.5 IU/mL HBs antigen was used as the sample.

In FIG. 12, an S/N ratio was determined during performing the method for detecting a test substance in Example 2 from the current when FBS (concentration of HBs antigen: 0 IU/mL) was used as the sample and the current when FBS containing 12.5 IU/mL HBs antigen was used as the sample. As a result, the S/N ratio was 33.7.

### (Comparative Example 3) Method for Detecting Test Substance

The electrode substrate 30b on which the test substance was immobilized in Example 2 (5) was connected to a potentiostat. Next, 30 µL of 0.05 M hydrochloric acid was added dropwise so that the first working electrode 43, the second working electrode 44, the counter electrode 51 and the reference electrode 61 were covered. After completion of the dropwise addition, a potential of 2.1 V based on the reference electrode 61 was applied to both the first working electrode 43 and the second working electrode 44 for 15 seconds. Thereafter, a potential of -1.0 V based on the reference electrode 61 was applied to both the first working electrode 43 and the second working electrode 44 for 8 minutes. Thereafter, with respect to the first working electrode 43 and the second working electrode 44, the potential was swept between -0.4 V and 0.4 V by differential pulse voltammetry method, and the current flowing between the first working electrode 43 and the counter electrode 51 and the current flowing between the second working electrode 44 and the counter electrode 51 were measured.

The results of detecting the currents caused by the silver nanoparticle in the labeled complex bound to the test substance contained in the sample in Comparative Example 3 are shown in FIG. 12. In FIG. 12, the current detected by the method for detecting a test substance in Comparative Example 3 is the total of the current flowing between the first working electrode 43 and the counter electrode 51 and the current flowing between the second working electrode 44 and the counter electrode 51. In the figure, each of white bars indicates a current when FBS (concentration of HBs antigen: 0 IU/mL) was used as the sample, and each of hatched bars indicates a current when FBS containing 12.5 IU/mL HBs antigen was used as the sample.

In FIG. 12, an S/N ratio was determined during performing the method for detecting a test substance in Comparative Example 3 from the current when FBS (concentration of HBs antigen: 0 IU/mL) was used as the sample and the current when FBS containing 12.5 IU/mL HBs antigen was used as the sample. As a result, the S/N ratio was 6.8.

In the present comparative example, the same electrode substrate as in Example 2 was used. However, in the present comparative example, silver was deposited and ionized at both the first working electrode and the second working electrode. Therefore, the method for detecting a test substance in the present comparative example can be considered as being equivalent to a conventional method in which silver is deposited and ionized at only one working electrode having the total area of the area of the first working electrode and the area of the second working electrode. From the results of Example 2 and Comparative Example 3, it was found that, according to the method in Example 2, a signal equivalent to that of the conventional method is secured, and noise can be reduced as compared with the conventional method.

### (Comparative Example 4) Method for Detecting Test Substance

The electrode substrate 30b on which the test substance was immobilized in Example 2 (5) was connected to a potentiostat. Next, 30 µL of 0.05 M hydrochloric acid was added dropwise so that the first working electrode 43, the second working electrode 44, the counter electrode 51 and the reference electrode 61 were covered. After completion of the dropwise addition, a potential of 2.1 V based on the reference electrode 61 was applied to the first working electrode 43 for 15 seconds. Thereafter, a potential of -1.0 V based on the reference electrode 61 was applied to the first working electrode 43 for 8 minutes. Thereafter, with respect to the first working electrode 43, the potential was swept between -0.4 V and 0.4 V by differential pulse voltammetry method, and the current flowing between the first working electrode 43 and the counter electrode 51 was measured.

The results of detecting the currents caused by the silver nanoparticle in the labeled complex bound to the test substance contained in the sample in Comparative Example 4 are shown in FIG. 12. In the figure, each of white bars indicates a current when FBS (concentration of HBs antigen: 0 IU/mL) was used as the sample, and each of hatched bars indicates a current when FBS containing 12.5 IU/mL HBs antigen was used as the sample.

In FIG. 12, an S/N ratio was determined during performing the method for detecting a test substance in Comparative Example 4 from the current when FBS (concentration of HBs antigen: 0 IU/mL) was used as the sample and the current when FBS containing 12.5 IU/mL HBs antigen was used as the sample. As a result, the S/N ratio was 13.2.

In the present comparative example, the same electrode substrate as in Example 2 was used. However, in the present comparative example, silver was deposited and ionized at only the first working electrode. Therefore, the method for detecting a test substance in the present comparative example can be considered as being equivalent to a conventional method in which silver is deposited and ionized at only one working electrode having the same area as the area of the first working electrode. From the results of Example 2 and Comparative Example 4, it was found that, according to the method in Example 2, noise is suppressed to be equal to that in the method in Comparative Example 4, and a signal higher than that in the method in Comparative Example 4 is obtained.

From the above results, it was found that, according to the method of Example 2, an S/N ratio higher than that of the methods in Comparative Example 3 and Comparative Example 4 is obtained.

### (Example 3) Method for Detecting Test substance (Quantitative Measurement) (1) Immobilization of Test Substance

An HBs antigen (manufactured by Sysmex Corporation, trade name: HISCL HBsAg calibrator, 2500 IU/mL) serving as a test substance was added to FBS so that the HBs antigen had a concentration of 0.09, 0.19, 0.78, 3.12, 12.5 or 50 IU/mL to obtain HBs antigen-containing FBS. Next, FBS (concentration of HBs antigen: 0 IU/mL) or the HBs antigen-containing FBS (concentration of HBs antigen: 0.09, 0.19, 0.78, 3.12, 12.5 or 50 IU/mL) serving as a sample was mixed with the solution containing the labeled complex obtained in Example 2 (2) so that [sample]/[labeled complex solution] (volume ratio) was 3/1.

Four µL of the resulting mixed solution was added dropwise onto the surface of each of the first working electrode 43 and the second working electrode 44 on the electrode substrate 30b obtained in Example 2 (3). Thereafter, the electrode substrate 30b was allowed to stand still at room temperature for 45 minutes under high humidity conditions, thereby causing an antigen-antibody reaction. Next, the solution on the surface of each of the first working electrode 43 and the second working electrode 44 was removed. Four µL of phosphate buffered saline was added dropwise onto the surface of each of the first working electrode 43 and the second working electrode 44, and washing was performed by pipetting. After the washing, the phosphate buffered saline on the surface of each of the first working electrode 43 and the second working electrode 44 was removed. Each of the first working electrode 43 and the second working electrode 44 was washed with use of 1.5 mL of purified water and then air-dried. Accordingly, the test substance was immobilized on the surface of each of the first working electrode 43 and the second working electrode 44 on the electrode substrate 30b.

### (2) Electrochemical Measurement

The same operation as in Example 2 (6) was carried out, and the current flowing between the first working electrode 43 and the counter electrode 51 was measured.

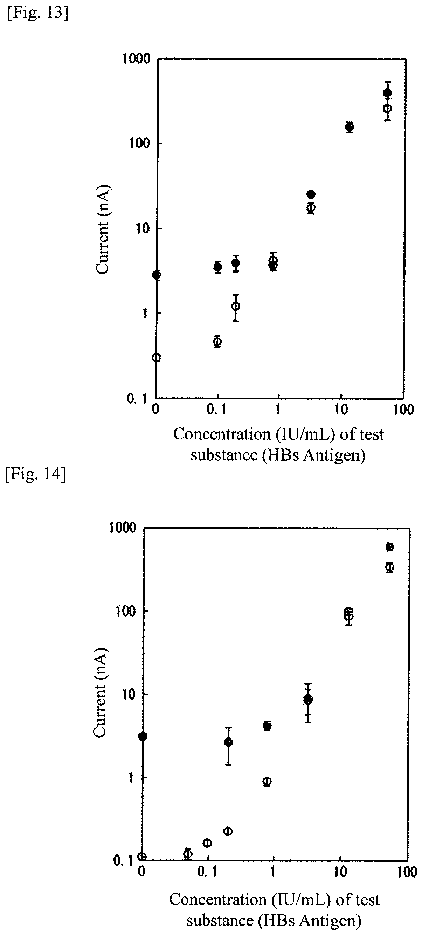

The results of examining the relationship between the concentration of the test substance in the sample and the current caused by the silver nanoparticle in the labeled complex bound to the test substance contained in the sample in Example 3 are shown in FIG. 13. In the figure, white circles indicate the results of measuring each current caused by the silver nanoparticle in the labeled complex bound to the test substance contained in the sample with use of the method for detecting a test substance in

### Example 3.

### (Comparative Example 5) Method for Detecting Test substance (Quantitative Measurement)

The electrode substrate 30b on which the test substance was immobilized in Example 3 (1) was connected to a potentiostat. Next, 30 µL of a 0.05 M aqueous hydrochloric acid solution was added dropwise so that the first working electrode 43, the second working electrode 44, the counter electrode 51 and the reference electrode 61 were covered. After completion of the dropwise addition, a potential of 2.1 V based on the reference electrode 61 was applied to both the first working electrode 43 and the second working electrode 44 for 15 seconds. Thereafter, a potential of -1.0 V based on the reference electrode 61 was applied to both the first working electrode 43 and the second working electrode 44 for 8 minutes. Thereafter, with respect to the first working electrode 43 and the second working electrode 44, the potential was swept between -0.4 V and 0.4 V by differential pulse voltammetry method, and the current flowing between the first working electrode 43 and the counter electrode 51 and the current flowing between the second working electrode 44 and the counter electrode 51 were measured.

The results of examining the relationship between the concentration of the test substance in the sample and the current caused by the silver nanoparticle in the labeled complex bound to the test substance contained in the sample in Comparative Example 5 are shown in FIG. 13. In FIG. 13, the current detected by the method for detecting a test substance in Comparative Example 5 is the total of the current flowing between the first working electrode 43 and the counter electrode 51 and the current flowing between the second working electrode 44 and the counter electrode 51. In the figure, black circles indicate the results of measuring each current caused by the silver nanoparticle in the labeled complex bound to the test substance contained in the sample by the method for detecting a test substance in Comparative Example 5.

As shown in FIG. 13, it is understood that, in the method for detecting a test substance in Example 3, even when a sample containing an HBs antigen with a low concentration (0.09 to 0.78 IU/mL) is used, noise is suppressed, a trace current derived from the HBs antigen with a low concentration can be quantitatively measured, and it is possible to quantitatively detect the HBs antigen with a low concentration. On the other hand, it is understood that, in the method for detecting a test substance in Comparative Example 5, quantitative measurement of an HBs antigen with a low concentration (0.09 to 0.78 IU/mL) is difficult. In FIG. 13, the detection limit in each detection method calculated from the current value + 3SD (standard deviation) with respect to the HBs antigen concentration: 0 IU/mL was 0.09 IU/mL in Example 3, and 0.78 IU/mL in Comparative Example 5. That is, the sensitivity of the detection method in Example 3 was about 8 times higher than the sensitivity of the detection method in Comparative Example 5.

In the present comparative example, the same electrode substrate as in Example 3 was used. However, in the present comparative example, silver was deposited and ionized at both the first working electrode and the second working electrode. Therefore, it can be considered as being equivalent to a conventional method in which silver is deposited and ionized at only one working electrode having the same area as the area of the first working electrode. From the results of Example 3 and Comparative Example 5, it was found that, according to the method in Example 3, even when a sample containing an HBs antigen with a low concentration is used, the detection sensitivity is improved more than the detection sensitivity when a conventional method for detecting a test substance is performed.

### (Example 4) Method for Detecting Test substance (Quantitative Measurement) (1) Immobilization of Primary Antibody

An anti-HBs antigen primary antibody solution was added to a sodium carbonate buffer (pH 9) so that the anti-HBs antigen primary antibody had a concentration of 100 µg/mL to obtain a primary antibody solution. Three µL of the resulting primary antibody solution was added dropwise onto the surface of each of the first working electrode 41 and the second working electrode 42 on the electrode substrate 30a obtained in Example 1 (1). Thereafter, the electrode substrate 30a was allowed to stand still at room temperature (25°C) for 2 hours under high humidity conditions. Next, the solution on the surface of each of the first working electrode 41 and the second working electrode 42 was removed. Four µL of phosphate buffered saline was added dropwise onto the surface of each of the first working electrode 41 and the second working electrode 42, and washing was performed by pipetting. After the washing, the phosphate buffered saline on the surface of each of the first working electrode 41 and the second working electrode 42 was removed.

### (2) Blocking Treatment

Four µL of 10% (w/w) BSA-containing phosphate buffered saline was added dropwise onto the surface of each of the first working electrode 41 and the second working electrode 42 on the electrode substrate main body to which the primary antibody was immobilized in Example 4 (1). Thereafter, the electrode substrate main body was allowed to stand still overnight at 4°C under high humidity conditions. Next, the solution on the surface of each of the first working electrode 41 and the second working electrode 42 was removed. Four µL of phosphate buffered saline was added dropwise onto the surface of each of the first working electrode 41 and the second working electrode 42, and washing was performed by pipetting. After the washing, the phosphate buffered saline on the surface of each of the first working electrode 41 and the second working electrode 42 was removed to obtain an electrode substrate 30c shown in FIG. 3(A).

### (3) Immobilization of Test Substance

An HBs antigen (manufactured by Sysmex Corporation, trade name: HISCL HBsAg calibrator, 2500 IU/mL) serving as a test substance was added to FBS so that the HBs antigen had a concentration of 0.09, 0.19, 0.78, 3.12, 12.5 or 50 IU/mL to obtain HBs antigen-containing FBS. Next, FBS (concentration of HBs antigen: 0 IU/mL) or the HBs antigen-containing FBS (concentration of HBs antigen: 0.09, 0.19, 0.78, 3.12, 12.5 or 50 IU/mL) serving as a sample was mixed with the solution containing the labeled complex obtained in Example 2 (2) so that [sample]/[labeled complex solution] (volume ratio) was 3/1.

Four µL of the resulting mixed solution was added dropwise onto the surface of each of the first working electrode 41 and the second working electrode 42 on the electrode substrate 30c obtained in Example 4 (2). Thereafter, the electrode substrate 30c was incubated while permeating the dropped solution at room temperature for 25 minutes under high humidity conditions, thereby performing an antigen-antibody reaction. Next, the solution on the surface of each of the first working electrode 41 and the second working electrode 42 was removed. Four µL of phosphate buffered saline was added dropwise onto the surface of each of the first working electrode 41 and the second working electrode 42, and washing was performed by pipetting. After the washing, the phosphate buffered saline on the surface of each of the first working electrode 41 and the second working electrode 42 was removed. Each of the first working electrode 41 and the second working electrode 42 was washed using 1.5 mL of purified water and then air-dried. Accordingly, the test substance was immobilized on the surface of each of the first working electrode 41 and the second working electrode 42 on the electrode substrate 30c.

### (4) Electrochemical Measurement

The electrode substrate 30c on which the test substance was immobilized in Example 4 (3) was connected to a potentiostat. Next, 30 µL of a 0.05 M aqueous hydrochloric acid solution was added dropwise so that the first working electrode 41, the second working electrode 42, the counter electrode 51 and the reference electrode 61 were covered. After completion of the dropwise addition, a potential of 2.1 V based on the reference electrode 61 was applied to both the first working electrode 41 and the second working electrode 42 for 15 seconds. Thereafter, a potential of -1.0 V based on the reference electrode 61 was applied only to the first working electrode 41 for 5 minutes. Thereafter, with respect to the first working electrode 41, the potential was swept between -0.4 V and 0.4 V by differential pulse voltammetry method, and the current flowing between the first working electrode 41 and the counter electrode 51 was measured.

The results of examining the relationship between the concentration of the test substance in the sample and each current caused by the silver nanoparticle in the labeled complex bound to the test substance contained in the sample in Example 4 are shown in FIG. 14. In the figure, white circles indicate the results of measuring each current caused by the silver nanoparticle in the labeled complex bound to the test substance contained in the sample by the method for detecting a test substance in Example 4.

### (Comparative Example 6) Method for Detecting Test substance (Quantitative Measurement)

The electrode substrate 30c on which the test substance was immobilized in Example 4 (3) was connected to a potentiostat. Next, 30 µL of a 0.05 M aqueous hydrochloric acid solution was added dropwise so that the first working electrode 41, the second working electrode 42, the counter electrode 51 and the reference electrode 61 were covered. After completion of the dropwise addition, a potential of 2.1 V based on the reference electrode 61 was applied to both the first working electrode 41 and the second working electrode 42 for 15 seconds. Thereafter, a potential of -1.0 V based on the reference electrode 61 was applied to both the first working electrode 41 and the second working electrode 42 for 5 minutes. Thereafter, with respect to both the first working electrode 41 and the second working electrode 42, the potential was swept between -0.4 V and 0.4 V by differential pulse voltammetry method, and the current flowing between the first working electrode 41 and the counter electrode 51 and the current flowing between the second working electrode 42 and the counter electrode 51 were measured.

The results of examining the relationship between the concentration of the test substance in the sample and each current caused by the silver nanoparticle in the labeled complex bound to the test substance contained in the sample in Comparative Example 6 are shown in FIG. 14. In the figure, black circles indicate the results of measuring each current caused by the silver nanoparticle in the labeled complex bound to the test substance contained in the sample by the method for detecting a test substance in Comparative Example 6.

From the results shown in FIG. 14, it is understood that, in the method for detecting a test substance in Example 4, even when a sample containing an HBs antigen with a low concentration (0.09 to 0.78 IU/mL) is used, noise is suppressed, a trace current derived from the HBs antigen with low concentration can be quantitatively measured, and it is possible to quantitatively detect the HBs antigen with low concentration, likewise the method for detecting a test substance in Example 3 (see white circles in FIG. 14). On the other hand, it is understood that, in the method for detecting a test substance in Comparative Example 6, quantitative measurement of the HBs antigen with a low concentration (0.09 to 0.78 IU/mL) is difficult.

In the present comparative example, the same electrode substrate as in Example 4 was used. However, in the present comparative example, silver was deposited and ionized at both the first working electrode and the second working electrode. Therefore, it can be considered as being equivalent to a conventional method in which silver is deposited and ionized at only one working electrode having the same area as the area of the first working electrode. From the results of Example 4 and Comparative Example 6, it was found that, according to the method in Example 4, even when a sample containing an HBs antigen with a low concentration is used, the detection sensitivity is improved more than the detection sensitivity when a conventional method for detecting a test substance is performed.

### (Example 5) Method for Detecting Test Substance

The detection sensitivity of a test substance was verified using an electrode substrate having an area ratio of a first working electrode and a second working electrode different from that of each of the electrode substrates used in Examples 1 to 4.

### (1) Preparation of Electrode Substrate

An electrode substrate main body having a first working electrode 45, a second working electrode 46, a counter electrode 51, and a reference electrode 61 was obtained in the same operation as in Example 1 (1), except for applying a carbon paste to a surface of the substrate main body 31 so as to have the patterns shown in FIG. 5, instead of applying the carbon paste to the surface of the substrate main body 31 so as to have the patterns shown in FIG. 3. In FIG. 5, the first working electrode 45 is integrally constituted with an electrode lead 71. In FIG. 5(B), the first working electrode 45 is an exposed portion that is not covered with a resist insulating film 80. In this portion, deposition of metal, ionization of metal and the like were performed. In FIG. 5(B), the first working electrode 45 is a portion denoted by A1 in FIG. 5(B). In addition, the second working electrode 46 is integrally constituted with an electrode lead 72. In FIG. 5(B), the second working electrode 46 is an exposed portion that is not covered with the resist insulating film 80. In this portion, ionization of the metal and the like were performed. In FIG. 5(B), the first working electrode 45 and the second working electrode 46 are hatched. An area of the first working electrode 45 [see A1 in FIG. 5(B)] was set to 0.45 mm². An area of the second working electrode 46 [see A2 in FIG. 5(B)] was set to 2.55 mm². [Area (A1) of first working electrode]/[Area of all working electrodes (A1 + A2)] was set to 3/20. A distance between the first working electrode 45 and the second working electrode 46 was set to 50 µm.

### (2) Immobilization of Primary Antibody

A primary antibody was immobilized on the electrode substrate main body obtained in Example 5 (1) in the same operation as in Example 2 (2), except for using the electrode substrate main body obtained in Example 5 (1) instead of using the electrode substrate main body obtained in Example 2 (1).

### (3) Blocking Treatment

An electrode substrate 30d was obtained in the same operation as in Example 2 (3), except for using the electrode substrate main body on which the primary antibody was immobilized in Example 5 (2) instead of using the electrode substrate main body on which a primary antibody was immobilized in Example 2 (2).

### (4) Immobilization of Test Substance

An HBs antigen (manufactured by Sysmex Corporation, trade name: HISCL HBsAg calibrator, 2500 IU/mL) serving as a test substance was added to FBS so that the HBs antigen had a concentration of 3.13 IU/mL to obtain HBs antigen-containing FBS. Next, FBS (concentration of HBs antigen: 0 IU/mL) or the HBs antigen-containing FBS (concentration of HBs antigen: 3.13 IU/mL) serving as a sample was mixed with the solution of the labeled complex obtained in Example 2 (2) so that [sample]/[labeled complex solution] (volume ratio) was 1/1.

Four µL of the resulting mixed solution was added dropwise onto the surface of each of the first working electrode 45 and the second working electrode 46 on the electrode substrate 30d obtained in Example 5 (3). Thereafter, the electrode substrate was allowed to stand still at room temperature for 90 minutes under high humidity conditions, thereby performing an antigen-antibody reaction. Next, the solution on the surface of each of the first working electrode 45 and the second working electrode 46 was removed. Four µL of phosphate buffered saline was added dropwise onto the surface of each of the first working electrode 45 and the second working electrode 46, and washing was performed by pipetting. After the washing, the phosphate buffered saline on the surface of the first working electrode 45 and the second working electrode 46 was removed. Each of the first working electrode 45 and the second working electrode 46 was washed using 1.5 mL of purified water and then air-dried. Accordingly, the test substance was immobilized on the surface of each of the first working electrode 45 and the second working electrode 46 on the electrode substrate 30d.

### (5) Electrochemical Measurement

The electrode substrate 30d on which the test substance was immobilized in Example 5 (4) was connected to a potentiostat. Next, 30 µL of a 0.05 M aqueous hydrochloric acid solution was added dropwise so that the first working electrode 45, the second working electrode 46, the counter electrode 51 and the reference electrode 61 were covered. After completion of the dropwise addition, a potential of 2.1 V based on the reference electrode 61 was applied to both the first working electrode 45 and the second working electrode 46 for 30 seconds. Thereafter, a potential of -1.0 V based on the reference electrode 61 was applied only to the first working electrode 45 for 4 minutes. Thereafter, with respect to the first working electrode 45, the potential was swept between -0.4 V and 0.4 V by differential pulse voltammetry method, and the current flowing between the first working electrode 45 and the counter electrode 51 was measured.

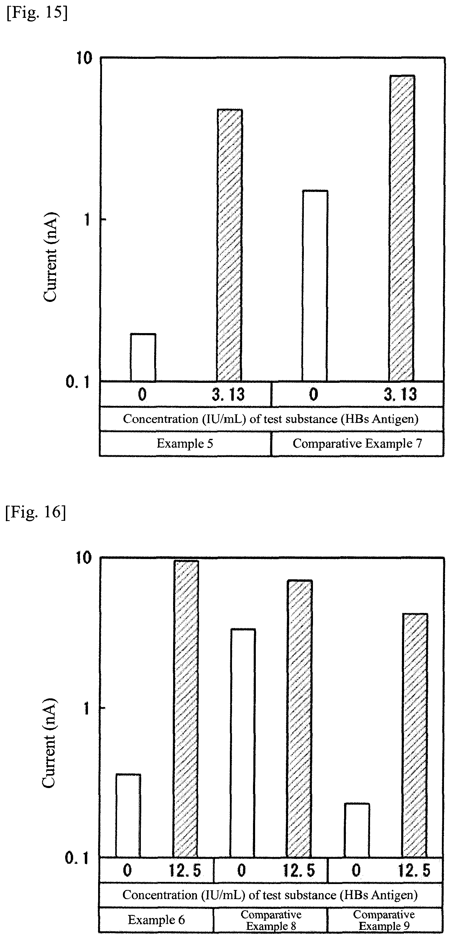

The results of detecting the currents caused by the silver nanoparticle in the labeled complex bound to the test substance contained in the sample in Example 5 are shown in FIG. 15. In the figure, each of white bars indicates a current when FBS (concentration of HBs antigen: 0 IU/mL) was used as the sample, and each of hatched bars indicates a current when FBS containing 3.13 IU/mL HBs antigen was used as the sample.

In FIG. 15, an S/N ratio was determined during performing the method for detecting a test substance in Example 5 from the current when FBS (concentration of HBs antigen: 0 IU/mL) was used as the sample and the current when FBS containing 3.13 IU/mL HBs antigen was used as the sample. As a result, the S/N ratio was 23.5.

### (Comparative Example 7) Method for Detecting Test Substance

The electrode substrate 30d on which the test substance was immobilized in Example 5 (4) was connected to a potentiostat. Next, 30 µL of a 0.05 M aqueous hydrochloric acid solution was added dropwise so that the first working electrode 45, the second working electrode 46, the counter electrode 51 and the reference electrode 61 were covered. After completion of the dropwise addition, a potential of 2.1 V based on the reference electrode 61 was applied to both the first working electrode 45 and the second working electrode 46 for 30 seconds. Thereafter, a potential of -1.0 V based on the reference electrode 61 was applied to both the first working electrode 45 and the second working electrode 46 for 4 minutes. Thereafter, with respect to the first working electrode 45, the potential was swept between -0.4 V and 0.4 V by differential pulse voltammetry method, and the current flowing between the first working electrode 45 and the counter electrode 51 and the current flowing between the second working electrode 46 and the counter electrode 51 were measured.

The results of detecting the currents caused by the silver nanoparticle in the labeled complex bound to the test substance contained in the sample in Comparative Example 7 are shown in FIG. 15. In FIG. 15, the current detected by the detection method of the test substance of Comparative Example 7 is the total of the current flowing between the first working electrode 45 and the counter electrode 51 and the current flowing between the second working electrode 46 and the counter electrode 51. In the figure, each of white bars indicates a current when FBS (concentration of HBs antigen: 0 IU/mL) was used as the sample, and each of hatched bars indicates a current when FBS containing 3.13 IU/mL HBs antigen was used as the sample.

As shown in FIG. 15, it is understood that, in the method for detecting a test substance in Example 5, noise derived from the electrode is low as compared with that in the method for detecting a test substance in Comparative Example 7, but a signal derived from the HBs antigen is the same level as that in the method for detecting a test substance in Comparative Example 7. In FIG. 15, an S/N ratio was determined during performing the method for detecting a test substance in Comparative Example 7 from the current when FBS (concentration of HBs antigen: 0 IU/mL) was used as the sample and the current when FBS containing 3.13 IU/mL HBs antigen was used as the sample. As a result, the S/N ratio was 4.1.

In the present comparative example, the same electrode substrate as in Example 5 was used. However, in the present comparative example, silver was deposited and ionized at both the first working electrode and the second working electrode. Therefore, the method for detecting a test substance in the present comparative example can be considered as being equivalent to a conventional method in which silver is deposited and ionized at only one working electrode having the total area of the area of the first working electrode and the area of the second working electrode. From the results of Example 5 and Comparative Example 7, it was found that, according to the method in Example 5, a signal equivalent to that of the conventional method is secured, and noise can be reduced as compared with the conventional method.

### (Example 6) Method for Detecting Test Substance

An electrode substrate 30e was obtained in the same operation as in Example 2, except for setting the distance between the first working electrode 43 and the second working electrode 44 to 100 µm instead of setting the distance between the first working electrode 43 and the second working electrode 44 to 50 µm. A measurement was performed in the same operation as in Example 2 using the resulting electrode substrate 30e.

The results of detecting the currents caused by the silver nanoparticle in the labeled complex bound to the test substance contained in the sample in Example 6 are shown in FIG. 16. In the figure, each of white bars indicates a current when FBS (concentration of HBs antigen: 0 IU/mL) was used as the sample, and each of hatched bars indicates a current when FBS containing 3.13 IU/mL HBs antigen was used as the sample.

In FIG. 16, an S/N ratio was determined during performing the method for detecting a test substance in Example 5 from the current when FBS (concentration of HBs antigen: 0 IU/mL) was used as the sample and the current when FBS containing 3.13 IU/mL HBs antigen was used as the sample. As a result, the S/N ratio was 25.9.

### (Comparative Example 8)

The electrode substrate 30e on which the test substance was immobilized in Example 6 was connected to a potentiostat. Next, 30 µL of a 0.05 M aqueous hydrochloric acid solution was added dropwise so that the first working electrode 43, the second working electrode 44, the counter electrode 51 and the reference electrode 61 were covered. After completion of the dropwise addition, a potential of 2.1 V based on the reference electrode 61 was applied to both the first working electrode 43 and the second working electrode 44 for 30 seconds. Thereafter, a potential of -1.0 V based on the reference electrode 61 was applied to both the first working electrode 43 and the second working electrode 44 for 4 minutes. Thereafter, with respect to the first working electrode 43, the potential was swept between -0.4 V and 0.4 V by differential pulse voltammetry method, and the current flowing between the first working electrode 43 and the second working electrode 44 and the current flowing between the first working electrode 43 and the counter electrode 51 were measured.

The results of detecting the currents caused by the silver nanoparticle in the labeled complex bound to the test substance contained in the sample in Comparative Example 8 are shown in FIG. 16. In FIG. 16, the current detected by the method for detecting a test substance in Comparative Example 7 is the total of the current flowing between the first working electrode 43 and the counter electrode 51 and the current flowing between the second working electrode 44 and the counter electrode 51. In the figure, each of white bars indicates a current when FBS (concentration of HBs antigen: 0 IU/mL) was used as the sample, and each of hatched bars indicates a current when FBS containing 3.13 IU/mL HBs antigen was used as the sample.

In FIG. 16, an S/N ratio was determined during performing the method for detecting a test substance in Comparative Example 8 was determined from the current when FBS (concentration of HBs antigen: 0 IU/mL) was used as the sample and the current when FBS containing 3.13 IU/mL HBs antigen was used as the sample. As a result, the S/N ratio was 1.1.

In the present comparative example, the same electrode substrate as in Example 6 was used. However, in the present comparative example, silver was deposited and ionized at both the first working electrode and the second working electrode. Therefore, the method for detecting a test substance in the present comparative example can be considered as being equivalent to a conventional method in which silver is deposited and ionized at only one working electrode having the total area of the area of the first working electrode and the area of the second working electrode. From the results of Example 6 and Comparative Example 8, it was found that, according to the method in Example 6, a signal equivalent to that of the conventional method is secured, and noise can be reduced as compared with the conventional method.

### (Comparative Example 9)

The electrode substrate 30e on which the test substance was immobilized in Example 6 was connected to a potentiostat. Next, 30 µL of a 0.05 M aqueous hydrochloric acid solution was added dropwise so that the first working electrode 43, the second working electrode 44, the counter electrode 51 and the reference electrode 61 were covered. After completion of the dropwise addition, a potential of 2.1 V based on the reference electrode 61 was applied only to the first working electrode 43 for 30 seconds. Thereafter, a potential of -1.0 V based on the reference electrode 61 was applied only to the first working electrode 43 for 4 minutes. Thereafter, with respect to the first working electrode 43, the potential was swept between -0.4 V and 0.4 V by differential pulse voltammetry method, and the current flowing between the first working electrode 43 and the counter electrode 51 was measured.

The results of detecting the currents caused by the silver nanoparticle in the labeled complex bound to the test substance contained in the sample in Comparative Example 9 are shown in FIG. 16. In the figure, each of white bars indicates a current when FBS (concentration of HBs antigen: 0 IU/mL) was used as the sample, and each of hatched bars indicates a current when FBS containing 3.13 IU/mL HBs antigen was used as the sample.

In FIG. 16, an S/N ratio was determined during performing the method for detecting a test substance in Comparative Example 9 from the current when FBS (concentration of HBs antigen: 0 IU/mL) was used as the sample and the current when FBS containing 3.13 IU/mL HBs antigen was used as the sample. As a result, the S/N ratio was 17.2.

In the present comparative example, the same electrode substrate as in Example 6 was used. However, in the present comparative example, silver was deposited and ionized at only the first working electrode. Therefore, the method for detecting a test substance in the present comparative example can be considered as being equivalent to a conventional method in which silver is deposited and ionized at only one working electrode having the same area as the area of the first working electrode. From the results of Example 6 and Comparative Example 9, it was found that, according to the method in Example 6, noise is suppressed to be equal to that in the method in Comparative Example 9, and a signal higher than that in the method in Comparative Example 9 is obtained.

From the above results, it was found that, according to the method of Example 2, an S/N ratio higher than that in the methods in Comparative Example 8 and Comparative Example 9 is obtained.

### REFERENCE SIGNS LIST

- 10: Detection device
- 11: Substrate receiving unit
- 12: Display
- 13: Electric measuring device
- 14: Power source
- 15: Conversion unit
- 16: Control unit
- 30: Electrode substrate
- 30a: Electrode substrate
- 30b: Electrode substrate
- 30c: Electrode substrate
- 30d: Electrode substrate
- 30e: Electrode substrate
- 31: Substrate main body
- 41: First working electrode
- 42: Second working electrode
- 43: First working electrode
- 44: Second working electrode
- 45: First working electrode
- 46: Second working electrode
- 51: Counter electrode
- 61: Reference electrode
- 61a: Reference electrode main body
- 71: Electrode lead
- 72: Electrode lead
- 73: Electrode lead
- 74: Electrode lead
- 80: Resist insulating film
- 91: Capture substance
- 100: Kit
- 101: Reagent bottle
- 102: Reagent bottle
- 103: Reagent bottle
- 201: Sample
- 202: Sample
- 205: Sample
- 206: Test substance
- 207: Contaminant
- 210: Labeling binding substance
- 211a: Silver ion
- 211b: Silver
- 212: Binding substance
- 215: Labeling complex
- 220: Labeling binding substance
- 221a: Gold ion
- 221b: Gold
- 222: Binding substance
- 225: Labeling complex

## Claims

1. A method for detecting a test substance, the method comprising:
an immobilization step of immobilizing a complex containing a test substance and a metal particle on a surface of a working electrode on an electrode substrate, the electrode substrate comprising the working electrode and a counter electrode;
an ionization step of applying an oxidation potential to the working electrode, so that metal ions are generated from the metal particle in the complex immobilized on the working electrode;
a deposition step of applying a reduction potential to a portion having an area smaller than an area of the portion to which an oxidation potential is applied in the working electrode, so that metal formed from the metal ions is deposited on a surface of a portion to which the reduction potential is applied; and
a measurement step of measuring current, voltage or charge caused by the metal deposited in the deposition step.

2. A method for detecting a test substance, the method comprising:
an immobilization step of immobilizing a complex containing a test substance and a metal particle on a surface of each of a first working electrode and a second working electrode on an electrode substrate, the electrode substrate comprising the first working electrode, the second working electrode and a counter electrode;
an ionization step of applying an oxidation potential to the first working electrode and the second working electrode, so that metal ions are generated from the metal particle in the complex immobilized on each of the first working electrode and the second working electrode;
a deposition step of applying a reduction potential to the first working electrode, without applying a reduction potential to the second working electrode, so that metal formed from the metal ions is deposited on a surface of the first working electrode; and
a measurement step of measuring current, voltage or charge caused by the metal deposited in the deposition step.

3. The method according to claim 2, wherein in the measurement step, current, voltage or charge generated when metal ions is generated from the metal deposited on the surface of the first working electrode in the deposition step is measured; and
the test substance is detected based on the current, the voltage or the charge.

4. The method according to claim 2, wherein in the measurement step,
current, voltage or charge when the metal is deposited on the surface of the first working electrode in the deposition step is measured, or a change in current, a change in voltage or a change in charge accompanied with deposition of the metal on the surface of the first working electrode in the deposition step is measured, and
the test substance is detected based on the current, the voltage or the voltage upon deposition of the metal on the surface of the first working electrode or the change in current, the change in voltage or the change in charge accompanied with deposition of the metal.

5. The method according to any one of claims 1 to 4, wherein a binding substance that binds to the test substance is immobilized on the metal particle.

6. The method according to claim 5, wherein the binding substance is an antibody.

7. The method according to any one of claims 1 to 6, wherein a capture substance that binds to the test substance is immobilized on the surface of the working electrode.

8. The method of claim 7, wherein the capture substance is an antibody.

9. A method for detecting metal ions, the method comprising
a first deposition step of bringing a sample containing metal ions into contact with a surface of a working electrode on an electrode substrate, the electrode substrate comprising the working electrode and a counter electrode, and applying a reduction potential to the working electrode, so that metal formed from the metal ions is deposited on the surface of the working electrode;
an ionization step of applying an oxidation potential to the working electrode, so that metal ions are generated from the metal deposited in the first deposition step;
a second deposition step of applying a reduction potential to a portion having an area smaller than an area of the portion to which an oxidation potential is applied in the working electrode, so that the metal formed from the metal ions is deposited on a surface of the portion to which the reduction potential is applied; and
a measurement step of measuring current, voltage or charge caused by the metal deposited in the deposition step to measure the current, the voltage or the charge.

10. A method for detecting metal ions, the method comprising:
a first deposition step of bringing a sample containing metal ions into contact with a surface of each of working electrodes on an electrode substrate, the electrode substrate comprising a first working electrode, a second working electrode and a counter electrode, and applying a reduction potential to the working electrode, so that metal formed from the metal ions is deposited on the surface of each of the first working electrode and the second working electrode;
an ionization step of applying an oxidation potential to the first working electrode and the second working electrode, so that metal ions are generated from the metal deposited in the first deposition step;
a second deposition step of applying a reduction potential to the first working electrode, without applying a reduction potential to the second working electrode, so that the metal formed from the metal ions is deposited on the surface of the first working electrode; and
a measurement step of measuring current, voltage or charge caused by the metal deposited in the deposition step.

11. The method according to claim 10, wherein in the measurement step, current, voltage or charge generated when metal ions are generated from the metal deposited on the surface of the first working electrode in the deposition step is measured; and
the metal ions are detected based on the current, the voltage or the charge.

12. The method according to claim 10, wherein in the measurement step,
current, voltage or charge when the metal is deposited on the surface of the first working electrode in the deposition step is measured, or a change in current, a change in voltage or a change in charge accompanied with deposition of the metal on the first working electrode in the deposition step is measured, and
the metal ions are detected based on the current, the voltage or the charge upon deposition of the metal on the first working electrode or the change in current, the change in voltage or the change in charge accompanied with deposition of the metal.

13. The method according to any one of claims 9 to 12, wherein the metal is zinc, lead, copper, mercury, cadmium, gold, or silver.

14. An electrode substrate for use in the method for detecting a test substance according to any one of claims 1 to 8 or the method for detecting metal ions according to any one of claims 9 to 13, the electrode substrate comprising a working electrode and a counter electrode,
wherein the working electrode includes a portion for generating metal ions and a portion for depositing metal after generation of the metal ions, and is configured so that the portion for depositing the metal after generation of the metal ions is smaller than the portion for generating the metal ions.

15. An electrode substrate for use in the method for detecting a test substance according to any one of claims 1 to 8 or the method for detecting metal ions according to any one of claims 9 to 13, the electrode substrate comprising a working electrode and a counter electrode,
wherein the working electrode includes a first working electrode where an oxidation potential is applied and a reduction potential is applied after the application of the oxidation potential, and a second working electrode where an oxidation potential is applied but a reduction potential is not applied after the application of the oxidation potential.

16. A detection kit for a test substance, the kit comprising the electrode substrate according to claim 14 or 15, and a reagent containing metal particles.
